(19) Europäisches Patentamt
European Patent Office
Office européen des brevets

(11) Numéro de publication : **0 281 439 B1**

(12) # FASCICULE DE BREVET EUROPEEN

(45) Date de publication du fascicule du brevet : **12.06.91 Bulletin 91/24**

(21) Numéro de dépôt : **88400240.3**

(22) Date de dépôt : **02.02.88**

(51) Int. Cl.[5] : **C07C 69/743**, C07C 219/22, C07C 255/39, C07C 323/28, C07C 33/46, C07C 43/23, C07D 309/12, C07F 7/18, A01N 53/00, C07C 33/00, C07C 43/00

(54) **Nouveaux esters d'acides cyclopropanecarboxyliques apparentés à l'acide pyréthrique, leur procédé de préparation et leur application à la lutte contre les parasites.**

Le dossier contient des informations techniques présentées postérieurement au dépôt de la demande et ne figurant pas dans le présent fascicule.

(30) Priorité : **06.02.87 FR 8701458**

(43) Date de publication de la demande : **07.09.88 Bulletin 88/36**

(45) Mention de la délivrance du brevet : **12.06.91 Bulletin 91/24**

(84) Etats contractants désignés : **AT BE CH DE ES FR GB GR IT LI LU NL SE**

(56) Documents cités :
**EP-A- 0 041 021**
**EP-A- 0 050 534**

(73) Titulaire : **ROUSSEL-UCLAF**
**35, boulevard des Invalides**
**F-75007 Paris (FR)**

(72) Inventeur : **Cadiergue, Joseph**
**6, rue Jules Princet**
**F-93600 Aulnay Sous Bois (FR)**
Inventeur : **Demassey, Jacques**
**Allée Saint Jacques Chalifert**
**F-77144 Montevrain (FR)**
Inventeur : **Demoute, Jean Pierre**
**249 bis, rue de Rosny**
**F-93100 Montreuil Sous Bois (FR)**
Inventeur : **Tessier, Jean**
**30, rue Jean Moulin**
**F-94300 Vincennes (FR)**

(74) Mandataire : **Tonnellier, Marie-José et al**
**111, route de Noisy B.P. no 9**
**F-93230 Romainville (FR)**

EP 0 281 439 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne de nouveaux esters d'acides cyclopropanecarboxyliques apparentés à l'acide pyréthrique, leur procédé de préparation et leur application à la lutte contre les parasites.

L'invention a pour objet les composés de formule I

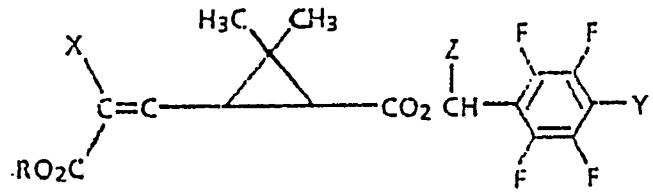

I

dans lesquels :

- X représente un atome d'hydrogène, de fluor, de chlore ou de brome,
- R représente un radical alkyle linéaire ramifié ou cyclique, saturé ou insaturé renfermant jusqu'à 8 atomes de carbone éventuellement substitué, un radical aryle renfermant jusqu'à 14 atomes de carbone éventuellement substitué ou un radical hétérocyclique éventuellement substitué
- Z représente un atome d'hydrogène, un radical $CH_3$, C≡N ou C≡CH, et
- Y représente un atome d'hydrogène, un radical hydroxy un radical alkyle linéaire, ramifié, saturé ou insaturé éventuellement substitué renfermant jusqu'à 8 atomes de carbone un radical nitrile, un radical $(CH_2)m$ O-alkyle, $(CH_2)m$ S alkyle $(CH_2)m$ N-(alkyle)$_2$, dans lequel m représente le nombre 0,1,2,3 ou 4 et renfermant jusqu'à 12 atomes de carbone un radical Si (alkyle)$_3$, alkyle représente un radical alkyle linéaire ou ramifié, saturé ou insaturé, éventuellement substitué renfermant jusqu'à 8 atomes de carbone, un radical –O– aryle ou $-(CH_2)_m-$ aryle, aryle représentant un radical aryle renfermant jusqu'à 14 atomes de carbone sous toutes les formes stéréoisomères possibles, ainsi que les mélanges de ces stéréoisomères.

Lorsque R représente un radical alcoyle saturé, linéaire ou ramifié il s'agit de préférence d'un radical méthyle, éthyle, n-propyle, isopropyle, n-butyle, sec-butyle, isobutyle, n-pentyle, n-hexyle, tert-butyle, tert-pentyle ou néopentyle.

Lorsque R représente un radical cyclique, il s'agit de préférence d'un radical cyclopropyle, cyclobutyle, cyclopentyle ou cyclohexyle, d'un radical alcoyle, linéaire ou ramifié, portant un radical cyclique ou d'un radical cyclopropyle, cyclobutyle, cyclopentyle ou cyclohexyle substitué par un ou plusieurs radicaux alcoyles dont la liaison avec le groupement –COO– est située sur l'un quelconque de ses sommets, par exemple, le radical 1-méthylcyclobutyle, 1-méthylcyclopentyle, 1-méthylcyclohexyle ou 2,2,3,3-tétraméthylcyclopropyle.

Lorsque R représente un radical alcoyle insaturé, il s'agit d'un radical éthylénique, comme par exemple, d'un radical vinyle ou 1,1-diméthylallyle ou d'un radical acétylénique, comme, par exemple, le radical éthynyle ou propynyle.

Lorsque R représente un radical alcoyle substitué par un ou plusieurs groupements fonctionnels, on entend de préférence par alcoyle un radical renfermant de 1 à 8 atomes de carbone, comme, par exemple, le radical méthyle, éthyle, propyle, isopropyle, n-butyle, isobutyle ou tertbutyle.

Lorsque R représente un radical alcoyle substitué par un ou plusieurs groupements fonctionnels, on entend de préférence par groupement fonctionnel, un atome d'halogène, un groupement OR' ou SR' dans lesquels R' représente un atome d'hydrogène ou un radical alcoyle renfermant de 1 à 8 atomes de carbone, un groupement :

$-NO_2$, 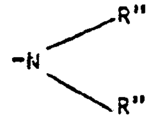

dans lequel R'' et R'', identiques ou différents, représentent un atome d'hydrogène, ou un radical alcoyle renfermant de 1 à 8 atomes de carbone, un groupement –C≡N, $SO_3H$ ou $PO_4H_2$ ou un groupement –COalc$_1$, $SO_2$alc$_2$, ou $SO_3$alc$_3$ dans lesquels alc$_1$, alc$_2$ et alc$_3$ représentent des radicaux alcoyles renfermant de 1 à 18 atomes de carbone.

R peut représenter également un radical alcoyle substitué par un radical aryle comme, par exemple, le radical benzyle ou le radical phénéthyle, lui-même éventuellement substitué par un ou plusieurs groupement –OH,

$-$Oalc ou alc renfermant de 1 à 8 atomes de carbone, par un ou plusieurs halogènes ou groupement $-CF_3$, $-OCF_3$, $-SCF_3$ ou par un groupement (G) :

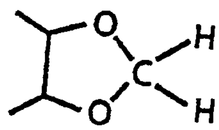

(G)

R peut représenter également un radical alcoyle substitué sur deux carbones adjacents par un groupement ($G_1$) :

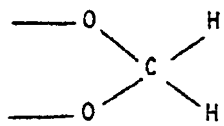

($G_1$)

substitué par un groupement

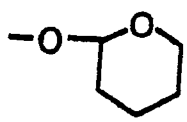

Lorsque R représente un radical alcoyle substitué par un ou plusieurs groupements fonctionels on peut citer comme valeurs préférées de R les radicaux :

$-(CH_2)_n$-C $Hal_3$ dans lequel n est un entier de 1 à 8 et Hal un atome d'halogène, par exemple le radical $-CH_2$-$CCl_3$, $-CH_2CF_3$,

$-CH_2$-$CH_2$-$CCl_3$ ou $-CH_2$-$CH_2$-$CF_3$,

$-(CH_2)_{n1}$ $-$CH $Hal_2$ dans lequel Hal est défini comme ci-dessus et $n_1$ est un nombre de 0 à 8, par exemple radical $-CH_2$-$CHCl_2$,

$-CH_2$-$CHF_2$ ou $-CHF_2$,

$-(CH_2)_n$ Hal dans lequel n et Hal sont définis comme ci-dessus, par exemple le radical $-CH_2$-$CH_2Cl$ ou $-CH_2$-$CH_2F$,

$-C$-$(CHal_3)_3$ dans lequel Hal est défini comme ci-dessus, par exemple un radical $-C$-$(CF_3)_3$ ou

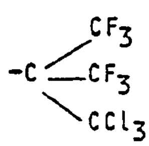

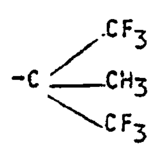 , 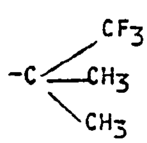 ou 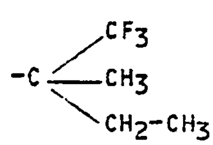

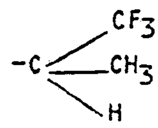 ou 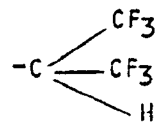

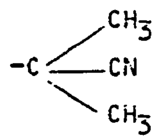 ou 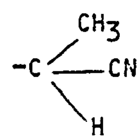

ou $-(CH_2)_n-CN$, dans lequel n est défini comme précédemment,

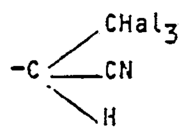

dans lequel Hal est défini comme précédemment, par exemple par le radical

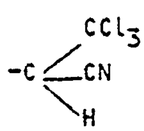

$-(CH_2)_n-OR'$, dans lequel n est défini comme précédemment et R' représente un atome d'hydrogène ou un radical alcoyle linéaire ou ramifié, comportant de 1 à 8 atomes de carbone, par exemple le radical $-CH_2-OCH_{3'}$, $-CH_2-CH_2-O-CH_{3'}$, $-CH_2CH_2-O-CH_2-CH_3$ ou $-CH_2-CH_2-OH$,

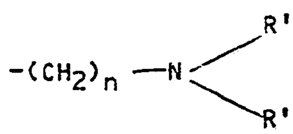

dans lequel n et R' sont définis comme précédemment et les deux radicaux R' peuvent être différents entre eux, par exemple le radical

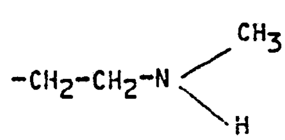

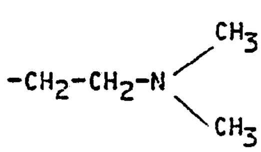 ou 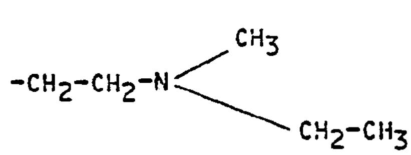

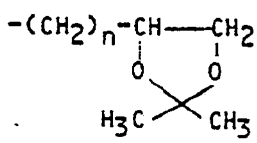

dans lequel n est défini comme précedemment, par exemple par le radical

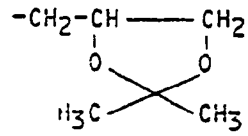

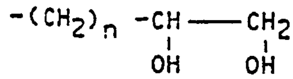

dans lequel n est défini comme précédemment, par exemple par le radical

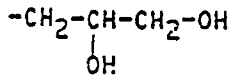

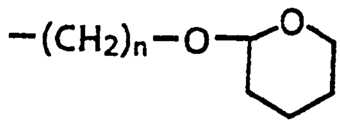

dans lequel n est défini comme précédemment, par exemple le radical

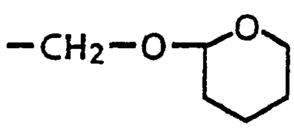

OU

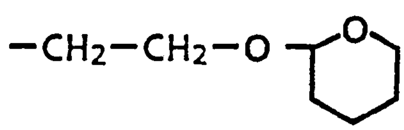

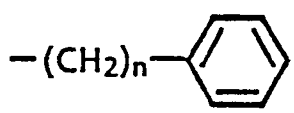

dans lequel n est défini comme précédemment, par exemple le radical benzyle ou phénéthyle,

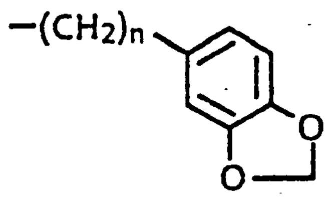

dans lequel n est défini comme précédemment, par exemple le radical

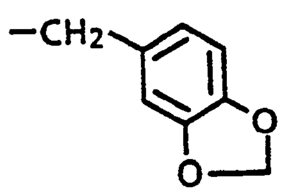

Lorsque R représente un radical aryle éventuellement substitué, il s'agit de préférence du radical phényle

ou du radical phényle substitué par un ou plusieurs groupements OH, Oalc alc renfermant de 1 à 8 atomes de carbone, *ou par un halogène ou un groupement* $-CF_3$, $-OCF_3$ *ou* $SCF_3$.

Lorsque R représente un radical hétérocyclique, il s'agit de préférence du radical pyridinyle, furanyle, thiophényle, oxazolyle ou thiazolyle.

Dans la définition de Y, lorsqu'il s'agit de radicaux alkyles, il s'agit de préférence de radicaux méthyle, éthyle, propyle, isopropyle, n-butyle ou tertbutyle.

Lorsque Y représente un radical alcoyle insaturé, il s'agit d'un radical éthylénique comme par exemple éthényle, propényle ou propadiényle ou d'un radical acétylinique comme par exemple le radical éthynyle ou propynyle.

Lorsque Y représente un radical alcoyle substitué par un ou plusieurs groupements fonctionnels, on entend de préférence par groupement fonctionnel les atomes d'halogène, tels que le fluor ou le brome.

Lorsque Y représente un radical O-aryle ou $(CH_2)_m$ aryle on entend de préférence par aryle le radical phényle.

L'invention a plus particulièrement pour objet les composés dans lesquels X représente un atome de fluor, et parmi ceux-ci, ceux dans lesquels la géométrie de la double liaison est E.

L'invention a également pour objet les composés de formule I, dans lesquels X représente un atome d'hydrogène, et parmi ceux-ci, ceux dans lesquels la géométrie de la double liaison est Z.

Parmi les composés préférés de l'invention, on peut citer les composés de formule I, dans lesquels R représente un radical alcoyle saturé linéaire ou ramifié renfermant jusqu'à 4 atomes de carbone, ainsi que ceux dans lesquels Z représente un atome d'hydrogène et ceux dans lesquels Y représente un radical $CH_3$, $(CH_2)_m$-O-$CH_3$ dans lequel m représente le nombre 0 ou 1 ou $CH_2$-CH=$CH_2$.

Parmi ceux-ci ont peut citer plus particulièrement les produits cités en exemples et tout particulièrement ceux dont les noms suivent :

- le 1R/1alpha, 3alpha (Z)/ 2,2-diméthyl 3-(3-méthoxy-3-oxo-1-propényl) cyclopropane carboxylate de (4-méthoxy 2,3,5,6-tétrafluorophényl) méthyle ;
- le 1R/1alpha,3alpha (E)/ 2,2-diméthyl 3-(2-fluoro-3-éthoxy-3-oxo-1-propényl) cyclopropane carboxylate de (4-méthyl-2,3,5,6-tétrafluorophényl) méthyle) ;
- le 1R/1alpha,3alpha (E)/ 2,2-diméthyl 3-[3-(1,1-diméthyl éthoxy) 3-oxo-2-fluoro-1-propényl] cyclopropane carboxylate de (4-méthyl-2,3,5,6-tétrafluorophényl) méthyle ;
- le [1R,(1alpha,3alpha)(E)]-2,2-diméthyl 3-(3-éthoxy 2-fluoro-3-oxo 1-propényl) cyclopropane carboxylate de [4-(2-propényl) 2,3,5,6-tétrafluorophényl] méthyle ;
- le 1R/1alpha,3alpha (E)/ 2,2-diméthyl 3-/2-fluoro 3-(1,1-diméthyl éthoxy-3-oxo-1-propényl/ cyclopropane carboxylate de (4-méthoxy 2,3,5, 6-tétrafluorophényl) méthyle ;
- le 1R/1alpha,3alpha (E)/ 2,2-diméthyl 3-(2-fluoro-3-éthoxy-3-oxo-1-propényl) cyclopropane carboxylate de (4-méthoxy-2,3,5,6-tétrafluorophényl) méthyle ;
- le [1R[1alpha,3alpha (E)]] 2,2-diméthyl 3-(2-fluoro-3-éthoxy-3-oxo-1-propényl) cyclopropane carboxylate de [(4-méthoxyméthyl-2,3,5,6-tétrafluoro) phényl] méthyle ;
- le [1R[1alpha,3alpha (E)]] 2,2-diméthyl 3-[3-(1,1-diméthyléthoxy 2-fluoro-3-oxo-1-propényl] cyclopropane carboxylate de [(4-méthoxyméthyl-2,3,5,6-tétrafluoro) phényl] méthyle ;
- le [1R[1alpha,3alpha (E)] 2,2-diméthyl 3-[2-fluoro-3-méthoxy-3-oxo-1-propènyl) cyclopropane carboxylate de [(4-méthoxyméthyl-2,3,5,6-tétrafluoro) phényl] méthyle.

Les composés de formule I présentent d'intéressantes propriétés qui permettent leur utilisation dans la lutte contre les parasites. Il peut s'agir par exemple de la lutte contre les parasites des végétaux, les parasites des locaux et les parasites des animaux à sang chaud.

C'est ainsi que l'on peut utiliser les produits de l'invention pour lutter contre les insectes, les nématodes et les acariens parasites des végétaux et des animaux.

L'invention a notamment pour objet l'application des composés de formule I à la lutte contre les parasites des végétaux, les parasites des locaux et les parasites des animaux à sang chaud.

Les produits de formule I peuvent donc être utilisés notamment pour lutter contre les insectes dans le domaine agricole, pour lutter par exemple, contre les pucerons, les larves de lépidoptères et les coléoptères, ainsi que pour lutter contre les insectes du sol. Ils sont utilisés à des doses comprises entre 10 g et 300 g de matière active à l'hectare.

Les produits de formule I peuvent également être utilisés pour lutter contre les insectes dans les locaux, pour lutter notamment contre les mouches, les moustiques et les blattes.

Les produits de formule I sont de plus photostables et ne sont pas toxiques pour les mammifères.

L'ensemble de ces propriétés fait des produits de formule I des produits qui correspondent parfaitement aux exigences de l'industrie agrochimique moderne : ils permettent de protéger les récoltes tout en préservant l'environnement.

Les produits de formule I peuvent aussi être utilisés pour lutter contre les acariens et les nématodes parasites des végétaux.

Les composés de formule I peuvent encore être utilisés pour lutter contre les acariens parasites des animaux, pour lutter par exemple contre les tiques et notamment les tiques de l'espèce de Boophilus, ceux de l'espèce Hyalomnia, ceux de l'espèce Amblyomnia et ceux de l'espèce Rhipicephalus ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits de formule I définis ci-dessus et notamment les produits de formule I.

L'invention a notamment pour objet les compositions insecticides renfermant comme principe actif au moins l'un des produits définis ci-dessus.

Ces compositions sont préparées selon les procédés usuels de l'industrie agrochimique ou de l'industrie vétérinaire ou de l'industrie des produits destinés à la nutrition animale.

Dans ces compositions destinées à l'usage agricole et à l'usage dans les locaux, la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005% à 10% en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un émanateur électrique.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 à 1% en poids.

Dans le cas où l'on utilise un support fibreux incombustible, la dose de matière active peut alors être, par exemple, de 0,03 à 95% en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95% en poids.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80% ou des liquides pour pulvérisation foliaire contenant de 1 à 500 g/l de principe actif. On peut également employer des poudres pour poudrages foliaires contenant de 0,05 à 3% de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo [2,2-1] 5-heptène-2,3-dicarboximide, ou le pipéronyl-bis-2-(2'-n-butoxy éthoxy) éthylacétal (ou tropital).

Les composés de formule I présentent une excellente tolérance générale, et l'invention a donc également

pour objet les produits de formule I, pour lutter notamment contre les affections créées par les tiques et les gales chez l'homme et l'animal.

Les produits de l'invention sont notamment utilisés pour lutter contre les poux à titre préventif ou curatif et pour lutter contre la gale.

Les produits de l'invention peuvent être administrés par voie externe, par vaporisation, par shampooing, par bain ou badigeonnage.

Les produits de l'invention à usage vétérinaire peuvent être également administrés par badigeonnage de l'épine dorsale selon la méthode dite méthode "pour-on".

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale I, et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcools 3-phénoxy benzylique et d'alcools alpha-cyano 3-phénoxy benzyliques des acides chrysanthémiques, par les esters d'alcools 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools alpha-cyano 3-phénoxy benzyliques des acides 2,2-diméthyl 3-(2,2-dichlorovinyl) cyclopropane-1-carboxyliques, par les esters d'alcools alpha-cyano 3-phénoxy benzyliques d'acides 2,2-diméthyl 3-(2,2-dibromovinyl) cyclopropane-1-carboxyliques, par les esters d'alcools 3-phénoxy benzylique des acides 2-parachlorophényl 2-isopropyl acétiques, par les esters d'alléthrolones, d'alcool 3,4,5,6-tétrahydrophtalimidométhylique, d'alcool 5-benzyl 3-furyl méthylique, d'alcool 3-phénoxy benzylique et d'alcools alpha-cyano 3-phénoxy benzyliques des acides 2,2-diméthyl 3-(1,2,2,2-tétrahaloéthyl) cyclopropane-1-carboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

L'invention a également pour objet un procédé de préparation des composés de formule I caractérisé en ce que l'on soumet un acide de formule II :

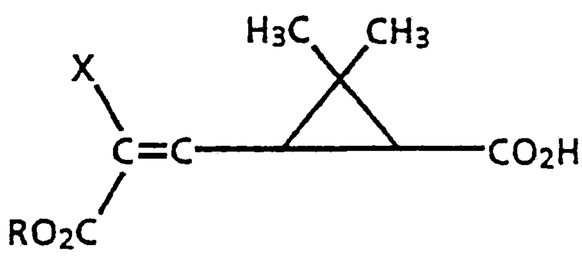

(II)

ou un dérivé fonctionnel de cet acide dans lequel X et R conservent leur signification précédente, à l'action d'un alcool de formule III :

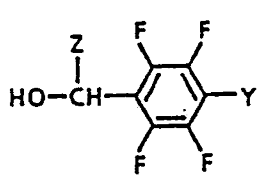

(III)

ou un dérivé fonctionnel de cet alcool dans lequel Y et Z conservent leur signification précédente pour obtenir le composé de formule I correspondant.

Les composés de formule II sont des produits connus décrits dans les brevets européens 0038271, 0041021, 0048186, 0050534.

Les composés de formule III dans lesquels Z représente un atome d'hydrogène sont des composés connus d'une manière générale ; ils peuvent être préparés par exemple selon les procédés décrits dans la demande de brevet européen 0031199, dans les brevets américains 4370346, 4405640, dans le brevet anglais 2171994 ou dans British Crop Protection Conference Pest and Desease 1986 page 199.

Les produits de formule III dans lesquels Z ne représente pas un atome d'hydrogène sont des produits

nouveaux. Ils peuvent être préparés selon le schéma réactionnel :

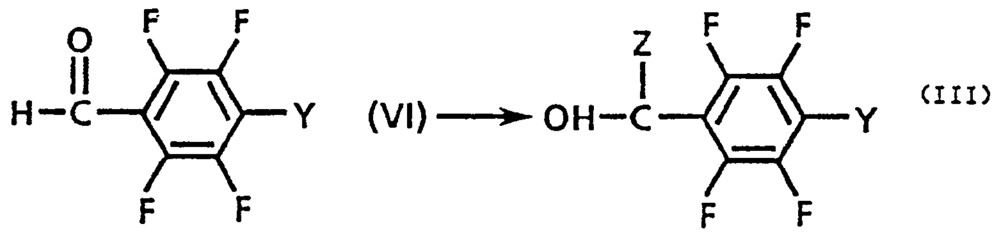

Lorsque l'on veut préparer un produit de formule III dans lequel Z représente un radical méthyle, on fait réagir le produit de formule VI avec un halogènure de méthylmagnésium, par exemple un iodure de méthylmagnésium.

Lorsque l'on veut préparer un produit de formule III dans lequel Z représente un radical C≡N, on fait réagir le produit de formule III avec un cyanure alcalin par exemple le cyanure de sodium ou de potassium.

Certains autres produits de formule III dont la préparation est donnée ci-après sont également des produits nouveaux et sont en eux-mêmes un objet de la présente invention.

L'invention a également pour objet une variante du procédé précédent caractérisé en ce que l'on soumet un acide de formule IV :

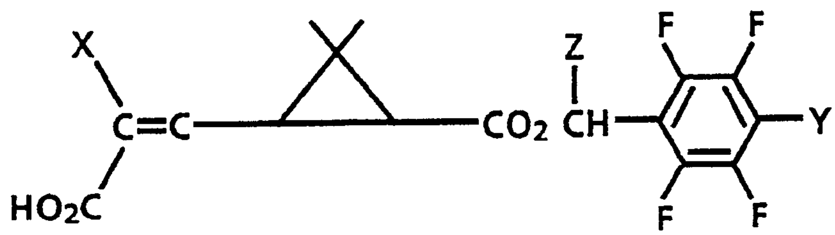

dans laquelle X, Y et Z conservent leur signification précédente, à l'action d'un alcool de formule V :

ROH (V)

dans laquelle R conserve sa signification précédente pour obtenir le composé de formule I correspondant.

Les exemples suivants illustrent l'invention sans toutefois la limiter.

**Exemple 1 : 1R/1alpha (R,S) 3alpha (E)/ 2,2-diméthyl 3-(2-fluoro-3-éthoxy-3-oxo-1-propényl) cyclopropane carboxylate de 1-(4-méthyl-2,3,5,6-tétrafluoro-phényl) éthyle**

On introduit à 0°C 950 mg de dicyclohexylcarbodiimide dans une solution renfermant 1 g d'acide 1R/1alpha (R,S) 3alpha (E)/ 2,2-diméthyl 3-(2-fluoro-3-éthoxy-3-oxo-1-propényl) cyclopropane carboxylique, 910 mg d'alcool alpha-méthyl 4-méthyl 2,3,5,6-tétrafluoro benzylique, 20 cm3 de chlorure de méthylène et 50 mg de 4-diméthylaminopyridine. On agite le mélange réactionnel pendant 5 heures à 0°C, élimine par filtration le précipité formé, et chasse le solvant sous pression réduite. On obtient 2,12 g d'un produit que l'on chromatographie sur silice en éluant par le mélange hexane-éther isopropylique (8-2). On obtient 1,6 g de produit recherché : $\alpha_D$ = +41° ± 1,5° (c = 1% $CHCl_3$).

**Exemples 2 à 20 :**

En opérant comme à l'exemple 1, en suivant le schéma réactionnel :

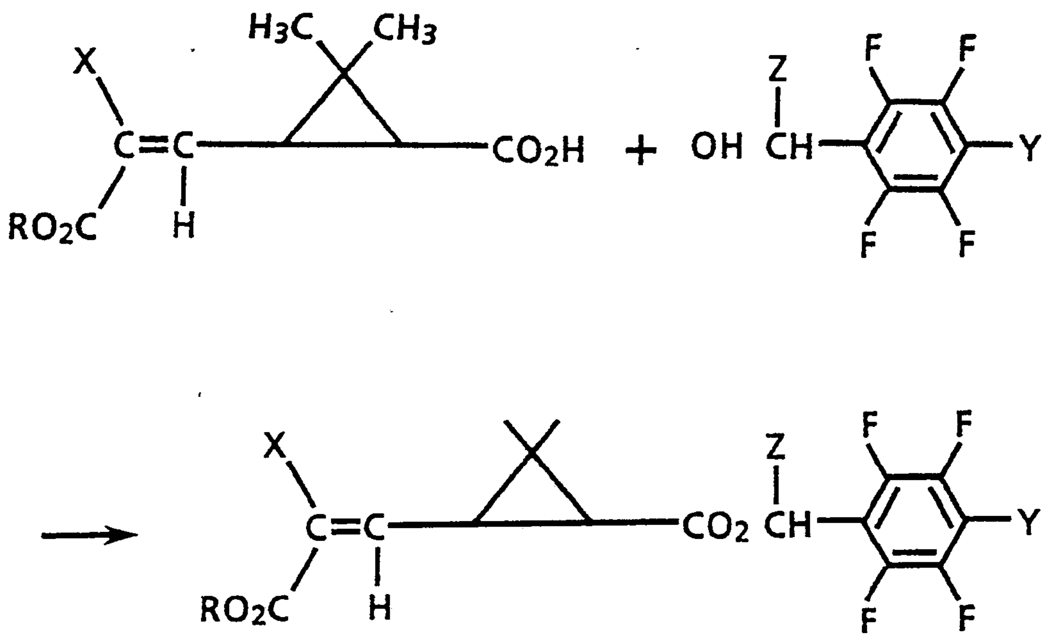

On a obtenu les produits suivants :

**Exemple 2 : 1R/1alpha (R,S) 3alpha (E)/ 2,2-diméthyl 3-[2-fluoro-3-(1,1-diméthyl éthoxy) 3-oxo-1-propényl] cyclopropane carboxylate de 1-(4-méthyl 2,3,5,6-tétrafluorophényl) éthyle**

$alpha_D$ = +50,5° ± 1° (c = 1,3% $CHCl_3$)

**Exemple 3 : 1R/1alpha (R,S) 3alpha (Z)/ 2,2-diméthyl 3-(3-méthoxy-3-oxo-1-propényl) cyclopropane carboxylate de 1-(4-méthyl 2,3,5,6-tétrafluorophényl) éthyle**

$Alpha_D$ = +58° ± 1° (c = 1,2% $CHCl_3$)

**Exemple 4 : 1R/1alpha (R,S) 3alpha (E)/ 2,2-diméthyl 3-(2-fluoro-3-éthoxy-3-oxo 1-propényl) cyclopropane carboxylate de cyano (4-méthyl-2,3,5,6-tétrafluorophényl) méthyle**

$Alpha_D$ = +31,2° ± 1° (c = 1,3% $CHCl_3$)

**Exemple 5 : 1R/1alpha (R,S) 3alpha/(Z) 2,2-diméthyl 3-(3-méthoxy-3-oxo-1-propényl)cyclopropane carboxylate de cyano (4-méthyl-2,3,5,6-tétra-fluorophényl) méthyle**

$Alpha_D$ = +39° ± 1,5° (c = 0,85% $CHCl_3$)

**Exemple 6 : 1R/1alpha (R,S) 3alpha (E)/ 2,2-diméthyl 3-[2-fluoro-3-(1,1-diméthyl-éthoxy)-3-oxo-1-propényl] cyclopropane carboxylate de cyano (4-méthyl 2,3,5,6-tétrafluorophényl) méthyle**

$Alpha_D$ = +19° ± 1° (c = 1% $CHCl_3$)

**Exemple 7 : 1R/1alpha, 3alpha (Z)/ 2,2-diméthyl-3-(3-méthoxy-3-oxo-1-propényl) cyclopropane carboxylate de (4-méthyl 2,3,5,6-tétrafluorophényl) méthyle**

$Alpha_D$ = +20° ± 1° (c = 0,9% $CHCl_3$)

**Exemple 8 : 1R/1alpha, 3alpha (E)/ 2,2-diméthyl-3-(2-fluoro-3-éthoxy-3-oxo-1-propényl) cyclopropane carboxylate de (4-méthyl 2,3,5,6-tétrafluorophényl) méthyle)**

$Alpha_D = +4° \pm 1°$ (c = 0,95% $CHCl_3$)

**Exemple 9 : 1R/1alpha, 3alpha (E)/ 2,2-diméthyl 3-[3-(1,1-diméthyl éthoxy)-3-oxo-2-fluoro-1-propényl] cyclopropane carboxylate de (4-méthyl-2,3,5,6-tétrafluorophényl) méthyle**

$Alpha_D = 19,2°$ (c = 1,3% $CHCl_3$)

**Exemple 10 : 1R/1alpha, 3alpha (Z)/ 2,2-diméthyl 3-(3-méthoxy-3-oxo-1-propényl) cyclopropane carboxylate de (4-méthoxy 2,3,5,6-tétrafluorophényl) méthyle**

$Alpha_D = +17° \pm 1°$ (c = 0,75% $CHCl_3$)

**Exemple 11 : 1R/1alpha, 3alpha (E)/ 2,2-diméthyl 3-[2-fluoro 3-(1,1-diméthyl éthoxy)-3-oxo-1-propényl] cyclopropane carboxylate de (4-méthoxy 2,3,5,6-tétrafluorophényl) méthyle**

F = 76°C $Alpha_D = +13° \pm 1,5°$ (c = 0,8% $CHCl_3$)

**Exemple 12 : 1R/1alpha, 3alpha (E)/ 2,2-diméthyl 3-(2-fluoro-3-éthoxy-3-oxo-1-propényl) cyclopropane carboxylate de (4-méthoxy 2,3,5,6-tétrafluorophényl) méthyle**

$Alpha_D = -1,5° \pm 1,5°$ (c = 0,75% $CHCl_3$)

**Exemple 13 : 1R/1alpha, 3alpha (E)/ 2,2-diméthyl 3-[3-(1,1-diméthyl éthoxy)-3-oxo-2-fluoro-1-propényl] cyclopropane carboxylate de 2,3,5,6-tétrafluorophényl méthyle**

$Alpha_D = 22,5° \pm 1°$ (c = 1% $CHCl_3$)

**Exemple 14 : 1R/1alpha, 3alpha (Z)/ 2,2-diméthyl 3-(3-oxo 3-méthoxy-1-propényl) cyclopropane carboxylate de 2,3,5,6-tétrafluorophényl méthyle**

F 50°C $Alpha_D = +27° \pm 1°$ (c = 0,9% $CHCl_3$)

**Exemple 15 : 1R/1alpha, 3alpha (E)/ 2,2-diméthyl 3-(3-oxo 3-éthoxy 2-fluoro-1-propényl) cyclopropane carboxylate de 2,3,5,6-tétrafluorophényl méthyle**

F = 50°C $Alpha_D = -21° \pm 1,5°$ (c = 0,7% $CHCl_3$)

**Exemple 16 : 1R/2alpha, 3alpha (Z)/ 2,2-diméthyl 3-(3-oxo-3-méthoxy-1-propényl) cyclopropane carboxylate de 4-diméthylamino 2,3,5,6-tétrafluorophényl méthyle**

$Alpha_D = +14° \pm 1,5°$ (c = 0,7% $CHCl_3$)

**Exemple 17 : 1R/1alpha, 3alpha (E)/ 2,2-diméthyl 3-[3-(1,1-diméthyl éthoxy)-2-fluoro-3-oxo-1-propényl] cyclopropane carboxylate de 4-diméthylamino 2,3,5,6-tétrafluorophényl méthyle**

$Alpha_D = +9° \pm 1,5°$ (c = 0,4% $CHCl_3$)

**Exemple 18 : 1R/1alpha, 3alpha (E)/ 2,2-diméthyl 3-(2-fluoro 3-oxo 3-éthoxy-1-propényl) cyclopropane carboxylate de 4-diméthylamino 2,3,5,6-tétrafluorophényl méthyle**

$Alpha_D = -2,5° \pm 2°$ (c = 0,6% $CHCl_3$)

**Exemple 19 : 1R/1alpha, 3alpha (E)/ 2,2-diméthyl 3-[3-(1,1-diméthyl éthoxy)-2-fluoro-3-oxo-1-propényl] cyclopropane carboxylate de 4-(2-propényl) 2,3,5,6-tétrafluorophényl méthyle**

$Alpha_D$ = +16° ± 1° (c = 1% $CHCl_3$)

**Exemple 20 : 1R/1alpha, 3alpha (Z)/ 2,2-diméthyl 3-(3-oxo 3-méthoxy 1-propényl cyclopropane carboxylate de 4-(2-propényl) 2,3,5,6-tétrafluorophényl méthyle**

$Alpha_D$ = +6,5° ± 1° (c = 1% $CHCl_3$)

**Exemple 21 : 1R/1alpha, 3alpha (Z)/ 2,2-diméthyl 3-/3-(1,1-diméthyl éthoxy)-3-oxo-1-propényl/ cyclopropane carboxylate de /(4-méthoxy 2,3,5,6-tétrafluoro) phényl/ méthyle**

F ≃60°C
$Alpha_D$ = +27° ± 1° (c = 1% $CHCl_3$)

**Exemple 22 : 1R/1alpha, 3alpha (E)/ 2,2-diméthyl 3-/2-fluoro 3-/2,2,2-(trifluoro méthyl) éthoxy/ 3-oxo-1-propényl/ cyclopropane carboxylate de /(4-méthoxy 2,3,5,6-tétrafluoro) phényl/ méthyle**

F = 69°C
$Alpha_D$ = −2° ± 1° (c = 1% $CHCl_3$)

**Exemple 23 : 1R/1alpha, 3alpha (Z)/ 2,2-diméthyl 3-/3-(2,2,2-trifluorométhyl/ 1-(trifluoro méthyl) éthoxy/ 3-oxo-1-propényl/ cyclopropane carboxylate de /(4-méthoxy 2,3,5,6-tétrafluoro) phényl/ méthyle**

F = 78°C
$Alpha_D$ = +5,5° ± 1° (c = 1% $CHCl_3$)

**Exemple 24 : 1R/1alpha, 3alpha (E)/ 2,2-diméthyl 3-(2-fluoro 3-méthoxy 3-oxo-1-propényl) cyclopropane carboxylate de /(4-méthoxy 2,3,5,6-tétrafluoro) phényl/ méthyle**

$Alpha_D$ = +5° ± 2° (c = 0,7% $CHCl_3$)

**Exemple 25 : 1R/1alpha, 3alpha (E)/ 2,2-diméthyl 3-/3-(1,1-diméthyl éthoxy) 2-fluoro 3-oxo-1-propényl/ cyclopropane carboxylate de /(4-éthoxy 2,3,5,6-tétrafluoro) phényl/ méthyle**

$Alpha_D$ = +24,5° ± 2° (c = 0,5% $CHCl_3$)

**Exemple 26 : 1R/1alpha, 3alpha (E)/ 2,2-diméthyl 3-(2-fluoro 3-méthoxy 3 oxo 1-propényl) cyclopropane carboxylate de (4-éthoxy 2,3,5,6 tétrafluoro phényl) méthyle**

$Alpha_D$ = +17° ± 2° (c = 0,4% $CHCl_3$)

**Exemple 27 : 1R/1alpha, 3alpha (E)/ 2,2-diméthyl 3-(2-fluoro 3-méthoxy 3 oxo-1-propényl) cyclopropane carboxylate de [(4-méthylthio 2,3,5,6-tétrafluoro) phényl] méthyle**

$Alpha_D$ = +1° ± 2° (c = 0,5% $CHCl_3$)

**Exemple 28 : 1R/1alpha, 3alpha (E)/ 2,2-diméthyl 3-(2-fluoro-3-éthoxy 3-oxo-1-propényl) cyclopropane carboxylate de [(4-méthylthio 2,3,5,6-tétrafluoro) phényl] méthyle**

$Alpha_D$ = +4,5° ± 2° (c = 0,6% $CHCl_3$)

**Exemple 29 : 1R/1alpha, 3alpha (E)/ 2,2-diméthyl 3-[2-fluoro 3-(1,1-diméthyl éthoxy) 3-oxo-1-propényl] cyclopropane carboxylate de [(4-méthylthio 2,3,5,6-tétrafluoro) phényl] méthyle**

$Alpha_D$ = +5° ± 2° (c = 0,5% $CHCl_3$)

**Exemple 30 : 1R/1alpha, 3alpha (E)/ 2,2-diméthyl 3-(2-fluoro 3-méthoxy-3 oxo 1-propényl) cyclopropane carboxylate de [(4-difluorométhoxy 2,3,5,6 tétrafluoro) phényl] méthyle**

$Alpha_D$ = +20° ± 1° (c = 1% $CHCl_3$)

**Exemple 31 : 1R/1alpha, 3alpha (E)/ 2,2-diméthyl 3-/3(1,1-diméthyl éthoxy 2-fluoro-3-oxo 1-propényl) cyclopropane carboxylate de [(4-difluorométhoxy 2,3,5,6 tétrafluoro) phényl] méthyle**

$Alpha_D$ = +22° ± 1° (c = 1% $CHCl_3$)

**Exemple 32 : 1R/1alpha, 3alpha (E)/ 2,2-diméthyl [3-(3-1,1-diméthyléthoxy 2-fluoro-3-oxo 1-propényl) cyclopropane carboxylate de [(4-bromodifluoro méthoxy 2,3,5,6-tétrafluoro) phényl] méthyle**

$Alpha_D$ = +16,5° ± 2° (c = 0,5% $CHCl_3$)

**Exemple 33 : 1R/1alpha, 3alpha (E)/ 2,2-diméthyl 3-(2-fluoro 3-méthoxy 3-oxo 1-propényl) cyclopropane carboxylate de [(4-bromodifluorométhoxy 2,3,5,6-tétrafluoro) phényl] méthyle**

$Alpha_D$ = +7° ± 1° (c = 1% $CHCl_3$)

**Exemple 34 : 1R/1alpha, 3alpha (E)/ 2,2-diméthyl 3-(2-fluoro 3-éthoxy 3-ox 1-propényl cyclopropane carboxylate de /(4-méthoxy méthyl 2,3,5,6-tétrafluoro) phényl/ méthyle**

$Alpha_D$ = +7,5° ± 1° (c = 1% $CHCl_3$)

**Exemple 35 : 1R/1alpha, 3alpha (Z)/ 2,2-diméthyl 3-/3-méthoxy 3-oxo 1-propényl/ cyclopropane carboxylate de /(4-méthoxy méthyl 2,3,5,6-tétrafluoro) phényl/ méthyle**

$Alpha_D$ = +23° ± 1° (c = 1% $CHCl_3$)

**Exemple 36 : 1R/1alpha, 3alpha (E)/ 2,2-diméthyl 3-/3-(1,1-diméthyl éthoxy) 2-fluoro 3-oxo 1-propényl/ cyclopropane carboxylate de /(4-méthoxy méthyl 2,3,5,6-tétrafluoro) phényl/ méthyle**

$Alpha_D$ = +19,5° ± 1° (c = 1% $CHCl_3$)

**Exemple 37 : 1R/1alpha, 3alpha (E)/ 2,2-diméthyl 3-(2-fluoro 3-méthoxy 3-oxo 1-propényl) cyclopropane carboxylate de /(4-méthoxy méthyl 2,3,5,6-tétrafluoro) phényl/ méthyle**

$Alpha_D$ = +7° ± 2° (c = 0,5% $CHCl_3$)

**Exemple 38 : 1R/1alpha, 3alpha (E)/ 2,2-diméthyl 3-(3-éthoxy 2-fluoro 3-oxo 1-propényl) cyclopropane carboxylate de //4-(2-propényl) 2,3,5,6-tétrafluoro/phényl/ méthyle**

$Alpha_D$ = +11,5° ± 2° (c = 0,5% toluène)

**Exemple 39 : 1R/1alpha, 3alpha (Z)/ 2,2-diméthyl 3-/3-/2,2,2-trifluoro-1-(trifluorométhyl) éthoxy/-3-oxo 1-propényl/ cyclopropane carboxylate de //4-(2-propényl) 2,3,5,6 tétrafluoro/ phényl/méthyle**

F = 58°C
$Alpha_D$ = +5,5° ± 1° (c = 1% $CHCl_3$)

**Exemple 40 : 1R/1alpha, 3alpha (E)/ 2,2-diméthyl 3-/2-fluoro 3-(1,1-diméthyl éthoxy) 3-oxo 1-propényl/ cyclopropane carboxylate de //4-(2-propynyl) 2,3,5,6-tétrafluoro/ phényl/ méthyle**

$Alpha_D$ = +16,5° ± 1° (c = 1% $CHCl_3$)

**Exemple 41 : 1R/1alpha, 3alpha (E)/ 2,2-diméthyl 3-(2-fluoro-3-méthoxy 3-oxo 1-propényl) cyclopropane carboxylate de //4-(2-propynyl) 2,3,5,6-tétrafluoro/ phényl/ méthyle**

$Alpha_D$ = +8° ± 1° (c = 1% $CHCl_3$)

**Exemple 42 : 1R/1alpha, 3alpha (E)/ 2,2-diméthyl 3-/2-fluoro-3-(1,1-diméthyl éthoxy) 3-oxo 1-propényl/ cyclopropane carboxylate de //4-(1,2-propadienyl) 2,3,5,6-tétrafluoro/ phényl/ méthyle**

$Alpha_D$ = +9,5° ± 1° (c = 1% $CHCl_3$)

**Exemple 43 : 1R/1alpha, 3alpha (E)/ 2,2-diméthyl 3-(2-fluoro 3-méthoxy 3-oxo 1-propényl) cyclopropane carboxylate de //4-(1,2-propadienyl) 2,3,5,6-tétrafluoro/ phényl/ méthyle**

$Alpha_D$ = +2° ± 1° (c = 1% $CHCl_3$)

**Exemple 44 : 1R/1alpha, 3alpha (E)/ 2,2-diméthyl 3-(2-fluoro 3-méthoxy 3-oxo 1-propényl) cyclopropane carboxylate de /(2,3,5,6 tétrafluoro) phényl/méthyle**

F = 83°C
$Alpha_D$ = +19,5° ± 1° (c = 1% toluène)

**Exemple 45 : 1R/1alpha, 3alpha (E)/ 2,2-diméthyl-3-/3-(1,1-diméthyl éthoxy) 2-fluoro 3-oxo-1-propényl/ cyclopropane carboxylate de /(4-cyano 2,3,5,6-tétrafluoro) phényl/ méthyle**

F = 87°C
$Alpha_D$ = +14° ± 1° (c = 1% $CHCl_3$)

**Exemple 46 : 1R/1alpha, 3alpha (E)/ 2,2-diméthyl-3-(2-fluoro 3-méthoxy-3-oxo 1-propényl) cyclopropane carboxylate de /(4-cyano 2,3,5,6-tétrafluoro) phényl/ méthyle**

$Alpha_D$ = +13° ± 1° (c = 1% $CHCl_3$)

**Exemple 47 : 1R/1alpha, 3alpha (E)/ 2,2-diméthyl 3-(3-éthoxy 2-fluoro 3-oxo propényl) cyclopropane carboxylate de /(4-phényl 2,3,5,6-tétrafluoro) phényl/méthyle**

F < 50°C
$Alpha_D$ = −12° ± 2° (c = 0,5% $CHCl_3$)

**Exemple 48 : 1R/1alpha, 3alpha (E)/ 2,2-diméthyl 3-/3(1,1-diméthyl éthoxy) 2-fluoro 3-oxo propényl/ cyclopropane carboxylate de /(4-phényl 2,3,5,6-tétrafluoro) phényl/ méthyle**

F = 90°C
$Alpha_D$ = −5,5° ± 1° (c = 1% $CHCl_3$)

**Exemple 49 : 1R/1alpha, 3alpha (E)/ 2,2-diméthyl 3-(2-fluoro 3-méthoxy 3-oxo-1-propényl) cyclopropane carboxylate de /(4-phénylméthyl) 2,3,5,6-tétrafluoro) phényl/ méthyle**

$Alpha_D$ = +5° ± 1° (c = 0,8% $CHCl_3$)

**Exemple 50 : 1R/1alpha, 3alpha (E)/ 2,2-diméthyl 3-/3(1,1-diméthyl éthoxy) 2-fluoro 3-oxo-1-propényl/ cyclopropane carboxylate de /(4-phénylméthyl 2,3,5,6-tétrafluoro) phényl/ méthyle**

F = 108°C

$Alpha_D = +7,5° \pm 1°$ (c = 1% $CHCl_3$)

**Exemple 51 : 1R/1alpha, 3alpha (Z)/ 2,2-diméthyl-3-/3-(2,2,2-trifluoro-1-trifluoro méthyl) éthoxy/ 3-oxo-1-propényl/ cyclopropane carboxylate de /(4-méthyl 2,3,5,6-tétrafluoro) phényl/ méthyle**

F < 50°C
$Alpha_D = +8° \pm 1°$ (c = 1% $CHCl_3$)

**Exemple 52 : 1R/1alpha, 3alpha (E)/ 2,2-diméthyl-3-(2-fluoro-3-méthoxy-3-oxo-1-propényl) cyclopropane carboxylate de /(4-méthyl 2,3,5,6-tétrafluoro) phényl/ méthyle**

$Alpha_D = +4° \pm 2°$ (c = 0,7% $CHCl_3$)

**Exemple 53 : 1R/1alpha, 3alpha (E)/ 2,2-diméthyl 3-(2-fluoro-3-butoxy-3-oxo-1-propényl) cyclopropane carboxylate de /(4-méthyl 2,3,5,6-tétrafluoro) phényl/ méthyle**

**Stade A**

1R/1alpha, 3alpha (E)/ 2,2-diméthyl 3-(2-fluoro 3-hydroxy-3-oxo-1-propényl) cyclopropane carboxylate de /(4-méthyl 2,3,5,6-tétrafluoro) phényl/ méthyle

On chauffe à 130°C pendant 1 heure 3,85 g de produit préparé comme à l'exemple 9 dans 38,5 $cm^3$ de toluène en présence de 0,385 g d'acide paratoluène sulfonique. On laisse revenir à température ambiante, lave à l'eau, sèche et élimine les solvants sous pression réduite, et obtient 3,75 g de produit attendu F ≃90-95°C.

**Stade B**

1R/1alpha, 3alpha (E)/ 2,2-diméthyl 3-(2-fluoro 3-butoxy 3-oxo 1-propényl) cyclopropane carboxylate de /(4-méthyl 2,3,5,6-tétrafluoro) phényl/ méthyle

En traitant le produit obtenu au Stade A par du butanol dans le chlorure de méthylène en présence de dicyclohexyl carbodimide et diméthylaminopyridine, on obtient le produit attendu.

$Alpha_D = -32,5° \pm 2°$ (c = 0,5% $CHCl_3$)
En opérant de la même manière, en utilisant l'alcool approprié, on a préparé les produits suivants.

**Exemple 54 : 1R/1alpha, 3alpha (E)/ 2,2-diméthyl 3-(2-fluoro-3-propoxy-3-oxo-1-propényl/ cyclopropane carboxylate de /(4-méthyl 2,3,5,6-tétrafluoro) phényl/ méthyle**

$Alpha_D = -19,5° \pm 2°$ (c = 0,4% $CHCl_3$)

**Exemple 55 : 1R/1alpha, 3alpha (E)/ 2,2-diméthyl 3-/2-fluoro-3-(1-méthyl) éthoxy-3-oxo-1-propényl/ cyclopropane carboxylate de /(4-méthyl 2,3,5,6-tétrafluoro) phényl/ méthyle**

$Alpha_D = -4° \pm 2°$ (c = 0,6% $CHCl_3$)

**Exemple 56 : 1R/1alpha, 3alpha (E)/ 2,2-diméthyl 3-/3-éthoxy 2-fluoro-3-oxo-1-propényl/ cyclopropane carboxylate de /(4-hydroxy 2,3,5,6-tétrafluoro) phényl/ méthyle**

**Stade A**

1R/1alpha, 3alpha (E)/ 2,2-diméthyl 3-(3-éthoxy-2-fluoro-3-oxo-1-propényl) cyclopropane carboxylate de //4-(diméthyl (1,1-diméthyl) éthyl silyloxy/ 2,3,5,6-tétrafluoro/ phényl/ méthyle.

On opère comme à l'exemple 1 à partir de l'acide et de l'alcool appropriés et obtient le produit attendu.
Spectre IR ($CHCl_3$)
Aromatique 1656-1515-1497 $cm^{-1}$
C = 0 1727 $cm^{-1}$

**Stade B**

1R/1alpha, 3alpha (E)/ 2,2-diméthyl 3-(3-éthoxy 2-fluoro-3-oxo-1-propényl) cyclopropane carboxylate de /(4-hydroxy 2,3,5,6-tétrafluoro) phényl/ méthyle

On refroidit à 4°C 1,8 g du produit obtenu au Stade A dans 5 $cm^3$ de tétrahydrofuranne et ajoute en 5 minutes 3,5 $cm^3$ d'une solution de fluorure de tétrabutylammonium dans le tétrahydrofuranne (1M). On agite 30 minutes, verse sur une solution aqueuse glacée de chlorure d'ammonium, extrait à l'éther, lave à l'eau, et élimine le solvant sous pression réduite. Après chromatographie sur silice (éluant hexane-acétate d'éthyle 1-1) on obtient le produit attendu.

$Alpha_D$ = +5,5° ± 1° (c = 0,8% $CHCl_3$)

**Préparation 1 : alcool alpha-méthyl-2,3,5,6-tétrafluoro benzylique**

On introduit en 30 minutes 3,2 $cm^3$ d'iodure de méthyle dans une suspension renfermant 1 g de magnésium et 30 $cm^3$ d'éther éthylique et laisse sous agitation pendant 30 minutes.

On refroidit à 0°C et introduit en 30 minutes 4 g de 4-méthyl 2,3,5,6-tétrafluorobenzaldéhyde et 100 $cm^3$ d'éther éthylique.

On agite le mélange réactionnel pendant 1 heure à 0°C et le verse dans 150 $cm^3$ d'une solution aqueuse saturée en chlorure d'ammonium. On extrait à l'éther, sèche les phases éthérées et amène à sec sous pression réduite. On chromatographie le produit brut obtenu sur silice en éluant par le mélange hexane-éther isopropylique (8-2). On obtient 3,85 g de produit recherché Rf = 0,10.

**Préparation 2 : Alcool alpha-cyano 4-méthyl 2,3,5,6-tétrafluorobenzylique**

On introduit 1,66 g de cyanure de sodium dans un mélange renfermant 4 g de 4 méthyl 2,3,5,6-tétrafluorobenzaldéhyde, 70 $cm^3$ d'alcool méthylique et 20 $cm^3$ d'eau, refroidit à 0°C.

On agite le mélange réactionnel pendant 1 heure à 0°C et le verse dans l'eau. On extrait à l'éther, sèche les phases éthérées et amène à sec sous pression réduite.

On obtient 4,54 g de produit recherché fondant à 124°C.

**Préparation 3 : 2,3,5,6-tétrafluoro 4-(hydroxy méthyl) benzonitrile**

**Stade A**

2,3,5,6-tétrafluoro 4-//(tétrahydropyran-2-yl) oxy/ méthyl/ benzaldéhyde.

On refroidit à –60°C 13 g de 2-/(2,3,5,6-tétrafluoro phényl) méthoxy tétrahydropyranne dans 200 $cm^3$ de tétrahydrofuranne et ajoute goutte à goutte en 30 minutes 35,1 $cm^3$ de butyllithium (1, 6M) dans l'hexane, agite 1 heure à –55°C, ajoute en 5 minutes 5 $cm^3$ de diméthylformamide en solution dans 10 $cm^3$ de tétrahydrofuranne, agite 1 heure et demie à cette température ajoute 50 $cm^3$ de solution aqueuse saturée en chlorure de sodium, extrait à l'éther isopropylique, sèche et élimine les solvants sous pression réduite. On recueille 14,4 g de produit attendu utilisé tel quel pour le stade suivant.

**Stade B**

2,3,5,6-tétrafluoro 4-(hydroxy méthyl) benzonitrile

On dissout dans 100 $cm^3$ de méthanol 11,7 g de l'aldehyde obtenu ci-dessus et 6,8 g d'acétate de sodium trihydraté puis ajoute 4,2 g de chlorure d'ammonium. On verse dans l'eau, extrait à l'isopropanol, sèche et élimine les solvants sous pression réduite. On récupère 13,6 g d'oxime F 130°C. On chauffe 4 heures au reflux le produit obtenu dans 400 $cm^3$ d'acétonitrile en présence de 1,6 g d'acétate cuivreux, refroidit à 40°C, concentre à sec, reprend avec 300 $cm^3$ d'isopropanol, lave à l'eau puis à l'eau salée, sèche et élimine les solvants sous pression réduite. On recueille 11,3 g de produit nitrilé. On ajoute 0,625 g d'acide paratoluène sulfonique à 12,5 g du produit préparé comme ci-dessus dans 62,5 $cm^3$ de méthanol, agite 1 heure à température ambiante, verse dans l'eau glacée, extrait à l'isopropanol, lave à l'eau, à l'eau salée, sèche et élimine les solvants sous pression réduite. On recueille 11,5 g de produit attendu F < 50°C.

**Préparation 4 : Alcool 4-[(1,1-diméthyl éthyl) diméthyl siloxy] 2,3,5 6-tétrafluoro benzylique**

**Stade A**

/1-(tétrahydro 2H-pyran 2-yl) oxy/ 2,3,5,6-tétrafluoro 4-[(2-tétrahydropyranyloxy) méthyl] phénol

On ajoute 8,4 g de potasse dans une solution comprenant 15,3 g du dérivé pyranylé de l'alcool pentafluorobenzylique dans 140 $cm^3$ de terbutanol et chauffe 4 heures et demie au reflux. On laisse revenir à température ambiante ajoute une solution aqueuse saturée en phosphate acide de sodium glacée extrait à l'acétate d'éthyle, lave à l'eau et à l'eau salée, sèche et élimine les solvants sous pression réduite. On obtient 16 g de produit brut que l'on chromatographie sur silice (éluant hexane-acétate d'éthyle 7-3) et obtient 10,78 g de produit attendu F = 90°C.

**Stade B**

2-[[4-(1,1-diméthyl éthyl) diméthyl siloxy] 2,3,5,6-tétrafluorophényl] méthoxy] tétrahydropyranne

On refroidit à +4°C 2,8 g de produit obtenu ci-dessus en solution dans 14 $cm^3$ de tétrahydrofuranne, ajoute 1,7 $cm^3$ de triéthylamine) puis ajoute en 15 mn une solution de 1,8 g de chlorure de terbutyldiméthylsilyle dans 6 $cm^3$ de tétrahydrofuranne. On agite 40 minutes, filtre, et élimine le solvant reprend le résidu dans l'isopropanol, filtre et recueille 4 g de produit attendu.

**Stade C**

Alcool 4-[(1,1-diméthyl éthyl) diméthyl siloxy] 2,3,5,6-tétrafluoro benzylique

On ajoute à température ambiante 0,1 g d'acide paratoluène sulfonique dans une solution comprenant 2 g de produit obtenu au Stade B dans 10 $cm^3$ de méthanol. On agite 1 heure, verse dans l'eau glacée, extrait à l'éther, et élimine les solvants sous pression réduite. Après chromatographie du résidu sur silice (éluant chlorure de méthylène) on obtient 1,16 g de produit attendu.

**Préparation 5 : l'alcool 4-bromodifluorométhoxy 2,3,5,6 tétrafluorobenzylique est préparé selon le schéma réactionnel suivant :**

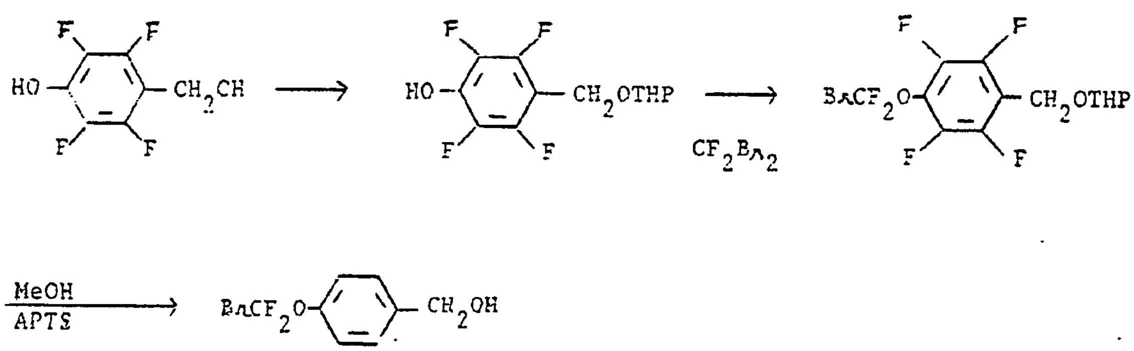

**Préparation 6 : l'alcool 4-difluorométhoxy 2,3,5,6-tétrafluorobenzylique est préparé selon le schéma *réactionnel suivant :***

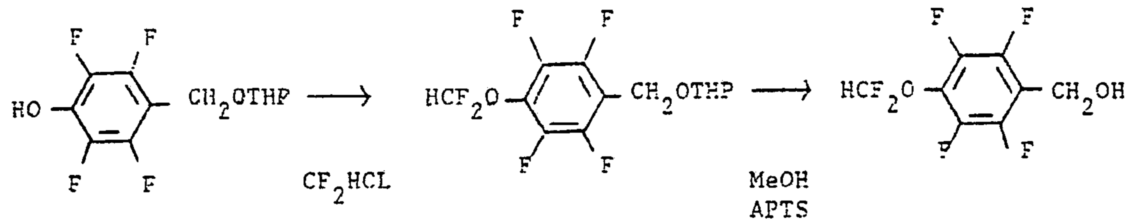


**Préparation 7 : le 2-[[2,3,5,6-tétrafluoro 4-(1,2-propa-diényl) phényl] méthoxy/tétrahydropyranne est préparé selon le schéma réactionnel suivant :**

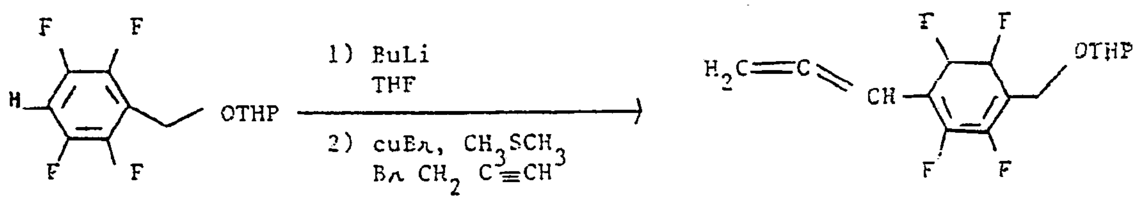


**Exemple 57 : Préparation d'un concentré soluble**

On effectue un mélange homogène de :

| | |
|---|---|
| Produit de l'exemple 8 : | 0,25 g |
| Butoxyde pipéronyle : | 1,00 g |
| Tween 80 : | 0,25 g |
| Topanol A : | 0,1 g |
| Eau : | 98,4 g |

**Exemple 58 : Préparation d'un concentré émulsifiable**

On mélange intimement :

| | |
|---|---|
| Produit de l'exemple 9 : | 0,015 g |
| Butoxyde de pipéronyle : | 0,5 g |
| Topanol A : | 0,1 g |
| Tween 80 : | 3,5 g |
| Xylène : | 95,885 g |

**Exemple 59 : Préparation d'un concentré émulsifiable**

On effectue un mélange homogène de :

| | |
|---|---|
| Produit de l'exemple 34 : | 1,5 g |
| Tween 80 : | 20,00 g |
| Topanol A : | 0,1 g |
| Xylène : | 78,4 g |

**Exemple 60 : Préparation d'une composition fumigène**

On mélange d'une façon homogène :

| | |
|---|---|
| Produit de l'exemple 36 : | 0,25 g |
| Poudre de tabu : | 25,00 g |
| Poudre de feuille de.cèdre : | 40,00 g |
| Poudre de bois de pin : | 33,75 g |
| Vert brillant : | 0,5 g |
| p-Nitrophénol : | 0,5 g |

ETUDE BIOLOGIQUE

A. Etude de l'effet d'abattage sur mouche domestique

Les insectes tests sont des mouches domestiques femelles âgées de 4 jours. On opère par pulvérisation directe à la concentration de 0,25 g/l en chambre de Kearns et March en utilisant comme solvant un mélange d'acétone (5%) et d'Isopar L (solvant pétrolier) (quantité de solvant utilisée 2 ml en une seconde). On utilise 50 insectes par traitement. On effectue les contrôles toutes les minutes jusqu'à 10 minutes, puis à 15 minutes et l'on détermine le KT 50 par les méthodes habituelles.

Les résultats expérimentaux obtenus sont résumés dans le tableau suivant :

| Composés | $KT_{50}$ en mn |
|---|---|
| Exemple 9 | 5,8 à 1 g/l |
| Exemple 8 | 6,5 à 100 mg/l |
| Exemple 34 | 1,54 à 100 mg/l |
| Exemple 36 | 2,611 à 1 g/l |
| Exemple 38 | 4,5 à 1 g/l |

B. Etude de l'effet létal des composés de l'invention sur divers insectes

a) Etude de l'effet létal sur mouche domestique

Les insectes tests sont des mouches domestiques femelles âgées de 4 à 5 jours. On opère par application topique de 1 l de solution acétonique sur le thorax dorsal des insectes à l'aide du micro manipulateur d'Arnold. On utilise 50 individus par traitement. On effectue le contrôle de mortalité vingt-quatre heures après traitement.

Les résultats obtenus exprimés en $DL_{50}$ ou dose (en nanogrammes) par individu nécessaire pour tuer 50% des insectes, sont les suivants :

| Composés | DL en ng/insecte |
|---|---|
| Exemple 9 | 25 |
| Exemple 8 | 19 |
| Exemple 34 | 8,3 |
| Exemple 36 | 13,7 |
| Exemple 38 | 3,34 |

b) Etude de l'effet létal sur blatte

Les tests sont efféctués par contact sur film sur verre, par dépôt à la pipette, de solutions acétoniques de différentes concentrations sur fond de boîte de Petri en verre dont les bords ont été préalablement talqués afin d'éviter la fuite des insectes. On détermine la concentration létale 50 (CL 50).

Les résultats expérimentaux obtenus sont résumés dans le tableau suivant :

| composés | CL 50 en mg/m2 |
|---|---|
| Exemple 9 | 0,22 |
| Exemple 8 | 0,33 |

c) Etude de l'effet létal sur larves de Spodoptera Littoralis

Les essais sont effectués par application topique d'une solution acétonique à l'aide du micro manipulateur d'Arnold sur le thorax dorsal des larves. On utilise 15 larves par dose de produit à tester. Les larves utilisées sont des larves du quatrième stade larvaire, c'est-à-dire âgées d'environ 10 jours lorsqu'elles sont élevées à 24°C et 65% d'humidité relative. Après traitement les individus sont placés sur milieu nutritif artificiel (milieu de Poitout).

On effectue le contrôle des mortalités 48 heures après traitement.

Les résultats expérimentaux obtenus sont résumés dans le tableau suivant :

| Composés | DL 50 en ng par insecte |
|---|---|
| Exemple 8 | 11,5 |

d) Etude de l'effet létal sur Aphis Cracivora

On utilise des adultes après 7 jours et l'on emploie 10 Aphis par concentration utilisée. On utilise une méthode de contact-ingestion. On effectue le traitement au pistolet de Fisher d'une feuille de fève que l'on dépose dans une boîte de Petri en matière plastique sur une rondelle de papier humidifiée. Le traitement est effectué à l'aide de 2 ml de solution acétonique de produit à tester (1 ml par face de feuille). L'infestation par insecte est effectuée après séchage de la feuille. On maintient les insectes en contact avec la feuille pendant une heure. On place les insectes sur des feuilles non traitées et contrôle la mortalité au bout de 24 heures.

Les résultats expérimentaux obtenus sont résumés dans le tableau suivant :

| Composés | DL 50 en ng/insecte |
|---|---|
| Exemple 9 | 0,5 |
| Exemple 8 | 1,6 |
| Exemple 34 | 0,716 |
| Exemple 36 | 1,665 |
| Exemple 38 | 0,485 |

C. ETUDE ACARICIDE DES COMPOSES DE L'INVENTION

On utilise des plants de haricot comportant 2 feuilles, infestées de 25 femelles de Tetranychus Urticae par feuille et mis sous bonette aérée sous plafond lumineux en lumière constante. Les plants sont traités au pistolet Fischer : 4 ml de solution toxique par plant d'un mélange à volume égal d'eau et d'acétone. On laisse sécher pendant 12 heures puis on procède à l'infestation. Les contrôles de mortalité sont effectués 80 heures après. La dose utilisée dans chaque test est de 5 g de produit par hl. On détermine la concentration létale 50 (CL 50).

Les résultats expérimentaux sont les suivants :

| Composés | CL 50 en mg/l |
|---|---|
| Exemple 9 | 75 |
| Exemple 8 | 500 |
| Exemple 34 | 244,3 |
| Exemple 36 | 2,278 |
| Exemple 38 | 185,4 |

**Revendications**

**Revendications pour les Etats Contractants : AT, BE, CH, DE, FR, GB, IT, LI, LU, NL, SE**

1. Les composés de formule I

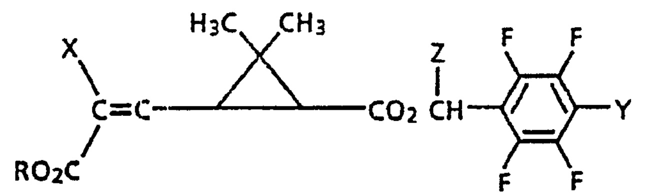

I

dans lesquels :

– X représente un atome d'hydrogène, de fluor, de chlore ou de brome,

– R représente un radical alkyle linéaire ramifié ou cyclique, saturé ou insaturé renfermant jusqu'à 8 atomes de carbone éventuellement substitué, un radical aryle renfermant jusqu'à 14 atomes de carbone éventuellement substitué ou un radical hétérocyclique éventuellement substitué

– Z représente un atome d'hydrogène, un radical $CH_3$, C≡N ou C≡CH, et

– Y représente un atome d'hydrogène, un radical hydroxy un radical alkyle linéaire, ramifié, saturé ou insaturé éventuellement substitué renfermant juqu'à 8 atomes de carbones un radical nitrile, un radical $-(CH_2)_m$-O-alkyle, $-(CH_2)_m$-S-alkyle, $-(CH_2)_m$-N (alkyle)$_2$, dans lequel m représente le nombre 0, 1, 2, 3 ou 4 et renfermant jusqu'à 12 atomes de carbone un radical Si(alkyle)$_3$, alkyle représente un radical alkyle linéaire ou ramifié, saturé ou insaturé, éventuellement substitué renfermant jusqu'à 8 atomes de carbone, un radical –O-aryle ou $(CH_2)_m$-aryle, aryle représentant un radical aryle renfermant jusqu'à 14 atomes de carbone sous toutes les formes stéréoisomères possibles, ainsi que les mélanges de ces stéréoisomères.

2. Les composés de formule I tels que définis à la revendication 1 dans lesquels X représente un atome de fluor.

3. Les composés de formule I tels que définis à la revendication 1 dans lesquels X représente un atome d'hydrogène.

4. Les composés de formule I tels que définis à l'une quelconque des revendications 1 à 3 dans lesquels

R représente un radical alcoyle saturé linéaire ou ramifié renfermant jusqu'à 4 atomes de carbone.

5. Les composés de formule I tels que définis à l'une quelconque des revendications 1 à 4 dans lesquels Z représente un atome d'hydrogène.

6. Les composés de formule I tels que définis à l'une quelconque des revendications 1 à 5 dans lesquels Y représente un radical $CH_3$, un radical $-(CH_2)_m-O-CH_3$ dans lequel m représente le nombre 0 ou 1 ou un radical $-CH_2-CH=CH_2$.

7. Les composés de formule I tels que définis à l'une quelconque des revendications 1 à 6 dont les noms suivent :

- le 1R/1alpha, 3alpha (Z)/ 2,2-diméthyl 3-(3-méthoxy-3-oxo-1-propényl) cyclopropane carboxylate de (4-méthoxy 2,3,5,6-tétrafluorophényl) méthyle ;
- le 1R/1alpha,3alpha (E)/ 2,2-diméthyl 3-(2-fluoro-3-éthoxy-3-oxo-1-propényl) cyclopropane carboxylate de (4-méthyl-2,3,5,6-tétrafluorophényl) méthyle) ;
- le 1R/1alpha,3alpha (E)/ 2,2-diméthyl 3-[3-(1,1-diméthyl éthoxy) 3-oxo-2-fluoro-1-propényl] cyclopropane carboxylate de (4-méthyl-2,3,5,6-tétrafluorophényl) méthyle ;
- le [1R,(1alpha,3alpha)(E)]-2,2-diméthyl 3-(3-éthoxy 2-fluoro-3-oxo 1-propényl) cyclopropane carboxylate de [4-(2-propényl) 2,3,5,6-tétrafluoro phényl] méthyle ;
- le 1R/1alpha,3alpha (E)/ 2,2-diméthyl 3-/2-fluoro 3-(1,1-diméthyl éthoxy-3-oxo-1-propényl/ cyclopropane carboxylate de (4-méthoxy 2,3,5, 6-tétrafluorophényl) méthyle ;
- le 1R/1alpha,3alpha (E)/ 2,2-diméthyl 3-(2-fluoro-3-éthoxy-3-oxo-1-propényl) cyclopropane carboxylate de (4-méthoxy-2,3,5,6-tétrafluorophényl) méthyle ;
- le [1R[1alpha,3alpha (E)]] 2,2-diméthyl 3-(2-fluoro-3-éthoxy-3-oxo-1-propényl) cyclopropane carboxylate de [(4-méthoxyméthyl-2,3,5,6-tétrafluoro) phényl] méthyle ;
- le [1R[1alpha,3alpha (E)]] 2,2-diméthyl 3-[3-(1,1-diméthyléthoxy 2-fluoro-3-oxo-1-propényl] cyclopropane carboxylate de [(4-méthoxyméthyl-2,3,5,6-tétrafluoro) phényl] méthyle ;
- le [1R[1alpha,3alpha (E)] 2,2-diméthyl 3-[2-fluoro-3-méthoxy-3-oxo-1-propènyl) cyclopropane carboxylate de [(4-méthoxyméthyl-2,3,5,6-tétrafluoro) phényl] méthyle.

8. Application des composés de formule I tels que définis à l'une quelconque des revendications 1 à 7, à la lutte contre les parasites des végétaux et les parasites des locaux.

9. Les compositions destinées à la lutte contre les parasites des végétaux, les parasites des locaux et les parasites des animaux à sang chaud, caractérisées en ce qu'elles renferment comme principe actif au moins un composé défini à l'une quelconque des revendications 1 à 7.

10. Les compositions insecticides, caractérisées en ce qu'elles renferment comme principe actif au moins un composé défini à l'une quelconque des revendications 1 à 7.

11. Les compositions acaricides, caractérisées en ce qu'elles renferment comme principe actif au moins un composé défini à l'une quelconque des revendications 1 à 7.

12. Associations douées d'activité insecticide, acaricide ou nématicide, caractérisées en ce qu'elles contiennent comme matière active, d'une part, un au moins des composés de formule générale I et ; d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools alpha-cyano 3-phénoxy benzyliques des acides 2,2-diméthyl 3-(2,2-dichlorovinyl) cyclopropane-1-carboxyliques, par les esters d'alcools alpha-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimidométhylique, d'alcool 5-benzyl 3-furyl méthylique, d'alcool 3-phénoxy benzylique et d'alcool alpha-cyano 3-phénoxy benzyliques des acides 2,2-diméthyl 3-(-1,2,2,2-tétrahaloéthyl) cyclopropane-1-carboxyliques, dans lesquels "halo" représente un atome de fluor, de chlore ou de brome, étant entendu que les composés I peuvent exister sous toutes leurs formes stéréoisomères possibles de même que les copules acides et alcools des esters pyréthrinoïdes ci-dessus.

13. Procédé de préparation des composés de formule I tel que défini à l'une quelconque des revendications 1 à 7 caractérisé en ce que l'on soumet un acide de formule II :

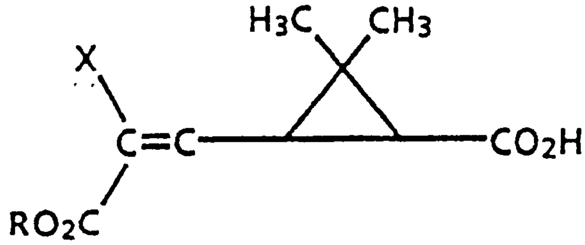

II

ou un dérivé fonctionnel de cet acide dans lequel X et R conservent leur signification précédente, à l'action d'un alcool de formule III :

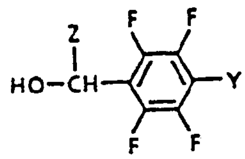

III

ou un dérivé fonctionnel de cet alcool dans lequel Y et Z conservent leur signification précédente pour obtenir le composé de formule I correspondant.

14. Variante du procédé de la revendication 13, caractérisé en ce que l'on soumet un acide de formule IV:

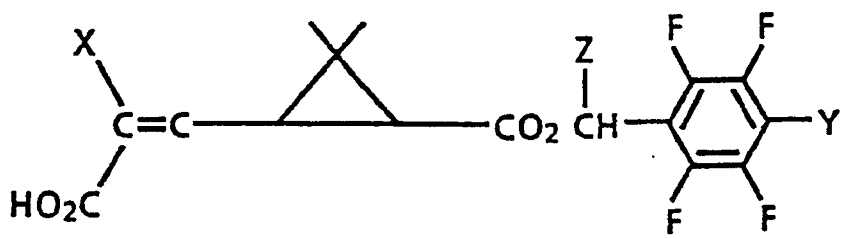

IV

dans laquelle X, Y et Z conservent leur signification précédente à l'action d'un alcool de formule V :

ROH (V)

dans laquelle R conserve sa signification précédente pour obtenir le composé de formule I correspondant.

15. A titre de produits chimiques nouveaux, les composés de formule III tels que définis à la revendication 13 dans lesquels Z ne représente pas un atome d'hydrogène.

16. A titre de produits chimiques nouveaux, les composés de formule III définis à la revendication 13 dont les noms suivent :

- l'alcool alpha-méthyl-4-méthyl-2,3,5,6-tétrafluorobenzylique ;
- l'alcool alpha-cyano-4-méthyl-2,3,5,6-tétrafluorobenzylique ;
- l'alcool alpha-méthyl-4-cyano-2,3,5,6-tétrafluorobenzylique ;
- l'alcool 4-[(1,1-diméthyl éthyl) diméthyl siloxy] 2,3,5,6 tétrafluorobenzylique ;
- l'alcool 4-bromo-difluorométhoxy-2,3,5,6-tétrafluorobenzylique ;
- l'alcool 4-difluorométhoxy-2,3,5,6-tétrafluorobenzylique ;
- 2-[[2,3,5,6-tétrafluoro 4-(1,2-propa-dienyl) phényl] méthoxy] tétrahydropyranne.

**Revendications pour l'Etat contractant suivant : ES**

1.Procédé pour préparer les composés de formule (I) :

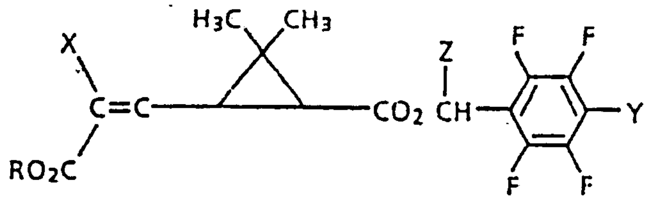

I

dans lesquels :

– X représente un atome d'hydrogène, de fluor, de chlore ou de brome,

– R représente un radical alkyle linéaire ramifié ou cyclique, saturé ou insaturé renfermant jusqu'à 8 atomes de carbone éventuellement substitué, un radical arylerenfermant jusqu'à 14 atomes de carbone éventuellement substitué ou un radical hétérocyclique éventuellement substitué

– Z représente un atome d'hydrogène, un radical $CH_3$, C≡N ou C≡CH,et

– Y représente un atome d'hydrogène, un radical hydroxy un radical alkyle linéaire, ramifié, saturé ou insaturé éventuellement substitué renfermant juqu'à 8 atomes de carbones un radical nitrile, un radical-$(CH_2)_m$-O-alkyle, –$(CH_2)_m$-S-alkyle,–$(CH_2)_m$-N (alkyle)$_2$, dans lequel m représente le nombre 0, 1, 2, 3 ou 4 et renfermant jusqu'à 12 atomes de carbone un radical Si(alkyle)$_3$, alkyle représente un radical alkyle linéaire ou ramifié, saturé ou insaturé, éventuellement substitué renfermant jusqu'à 8 atomes de carbone, un radical –O-aryle ou–$(CH_2)_m$-aryle, aryle représentant un radical aryle renfermant jusqu'à 14 atomes de carbone sous toutes les formes stéréoisomères possibles, ainsi que les mélanges de ces stéréoisomères, caractérisé en ce que l'on soumet un acide de formule (II) :

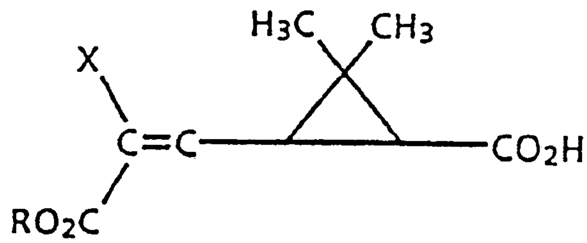

(II)

ou un dérivé fonctionnel de cet acide dans lequel X et R conservent leur signification précédente, à l'action d'un alcool de formule (III) :

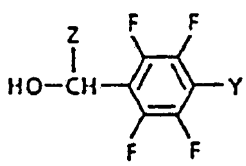

(III)

ou un dérivé fonctionnel de cet alcool dans lequel Y et Z conservent leur signification précédente pour obtenir le composé de formule (I) correspondant.

2. Procédé pour préparer les produits de formule (I) telle que définie à la revendication 1, caractérisé en ce que l'on soumet un acide de formule (IV) :

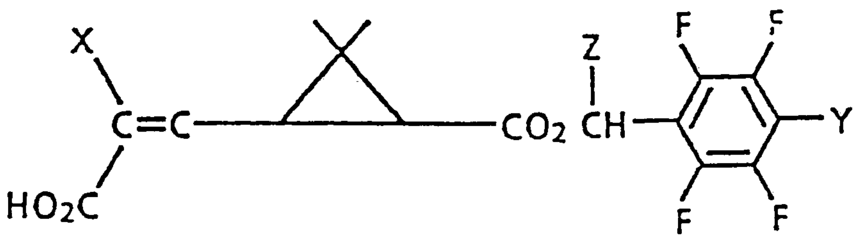

IV

dans laquelle X, Y et Z conservent leur signification précédente à l'action d'un alcool de formule V :

ROH (V)

dans laquelle R conserve sa signification précédente pour obtenir le composé de formule I correspondant.

3. Procédé selon la revendication 1, pour la préparation des produits de formule (I), telle que définie à la revendication 1, dans laquelle X, R et Z sont définis comme à la revendication 1 et Y représente un atome d'hydrogène, un radical alkyle linéaire, ramifié, saturé ou insaturé éventuellement substitué renfermant jusqu'à 8 atomes de carbone, un radical $(CH_2)_m$-O-alkyle, un radical $(CH_2)_m$-S-alkyle, un radical $(CH_2)_m(\text{alkyle})_2$ dans lesquels m représente le nombre 0, 1, 3 ou 4 et renfermant jusqu'à 12 atomes de carbone ou un radical Si(alkyle)$_3$ dans lequel le radical alkyle renfermant jusqu'à 8 atomes de carbone caractérisé en ce que l'on utilise au départ un produit de formule (II) dans laquelle X et R sont définis comme à la revendication 1 et un produit de formule (III) dans laquelle Z est défini comme à a revendication 1 et Y est défini comme ci-dessus.

4. Procédé selon la revendication 2, pour la préparation des produits de formule (I), telle que définie à la revendication 1, dans laquelle X, R et Z sont définis comme à la revendication 1 et Y représente un atome d'hydrogène, un radical alkyle linéaire, ramifié, saturé ou insaturé éventuellement substitué renfermant jusqu'à 8 atomes de carbone, un radical $(CH_2)_m$-O-alkyle, un radical $(CH_2)_m$-S-alkyle, un radical $(CH_2)_m(\text{alkyle})_2$ dans lesquels m représente le nombre 0, 1, 3 ou 4 et renfermant jusqu'à 12 atomes de carbone ou un radical Si(alkyle)3 dans lequel le radical alkyle renfermant jusqu'à 8 atomes de carbone, caractérisé en ce que l'on utilise au départ un produit de formule (V) dans lequel R est défini comme à la revendication 1 et un produit de formule (IV) dans laquelle X et Z sont définis comme à a revendication 1 et Y est défini comme ci-dessus.

5. Procédé selon la revendication 3, caractérisé en ce que l'on utilise au départ un produit de formule (II) dans laquelle X représente un atome de fluor.

6. Procédé selon la revendication 4, caractérisé en ce que l'on utilise au départ un produit de formule (IV) dans laquelle X représente un atome de fluor.

7. Procédé selon la revendication 3, caractérisé en ce que l'on utilise au départ un produit de formule (II) dans laquelle X représente un atome d'hydrogène.

8. Procédé selon la revendication 4, caractérisé en ce que l'on utilise au départ un produit de formule (IV) dans laquelle X représente un atome d'hydrogène.

9. Procédé selon l'une quelconque des revendications 3, 5 ou 7, caractérisé en ce que l'on utilise au départ un produit de formule (II) dans laquelle R représente un radical alcoyle saturé, linéaire ou ramifié renfermant jusqu'à 4 atomes de carbone.

10. Procédé selon l'une quelconque des revendications 4, 6 ou 8, caractérisé en ce que l'on utilise au départ un produit de formule (V) dans laquelle R représente un radical alcoyle saturé, linéaire ou ramifié renfermant jusqu'à 4 atomes de carbone.

11. Procédé selon l'une quelconque des revendications 3, 5, 7 ou 9, caractérisé en ce que l'on utilise au départ un produit de formule (III) dans laquelle R représente un atome d'hydrogène.

12. Procédé selon l'une quelconque des revendications 4, 6, 8 ou 10, caractérisé en ce que l'on utilise au départ un produit de formule (IV) dans laquelle R représente un atome d'hydrogène.

13. Procédé selon l'une quelconque des revendications 3, 5, 7, 9 ou 11, caractérisé en ce que l'on utilise au départ un produit de formule (III) dans laquelle Y représente un radical $CH_3$, un radical $-(CH_2)_m-O-CH_3$ dans lequel m représente le nombre 0 ou 1 un radical $-CH_2-CH=CH_2$.

14. Procédé selon l'une quelconque des revendications 4, 6, 8, 10 ou 12, caractérisé en ce que l'on utilise au départ un produit de formule (IV) dans laquelle Y représente un radical $CH_3$, un radical $-(CH_2)_m-O-CH_3$ dans lequel m représente le nombre 0 ou 1 ou un radical $-CH_2-CH=CH_2$.

**Revendications pour l'Etat contractant suivant : GR**

1. Procédé pour préparer les composés de formule (I) :

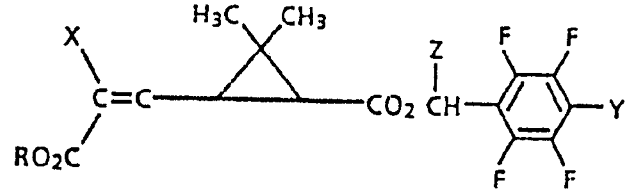

I

dans lesquels :

– X représente un atome d'hydrogène, de fluor, de chlore ou de brome,

– R représente un radical alkyle linéaire ramifié ou cyclique, saturé ou insaturé renfermant jusqu'à 8 atomes de carbone éventuellement substitué, un radical arylerenfermant jusqu'à 14 atomes de carbone éventuellement substitué ou un radical hétérocyclique éventuellement substitué

– Z représente un atome d'hydrogène, un radical $CH_3$, C≡N ou C≡CH,et

– Y représente un atome d'hydrogène, un radical hydroxy un radical alkyle linéaire, ramifié, saturé ou insaturé éventuellement substitué renfermant juqu'à 8 atomes de carbones un radical nitrile, un radical–$(CH_2)_m$-O-alkyle, –$(CH_2)_m$-S-alkyle, -$(CH_2)_m$-N (alkyle)$_2$, dans lequel m représente le nombre 0, 1, 2, 3 ou 4 et renfermant jusqu'à 12 atomes de carbone un radical Si(alkyle)$_3$, alkyle représente un radical alkyle linéaire ou ramifié, saturé ou insaturé, éventuellement substitué renfermant jusqu'à 8 atomes de carbone, un radical –O-aryle ou–$(CH_2)_m$-aryle, aryle représentant un radical aryle renfermant jusqu'à 14 atomes de carbone sous toutes les formes stéréoisomères possibles, ainsi que les mélanges de ces stéréoisomères, caractérisé en ce que l'on soumet un acide de formule (II) :

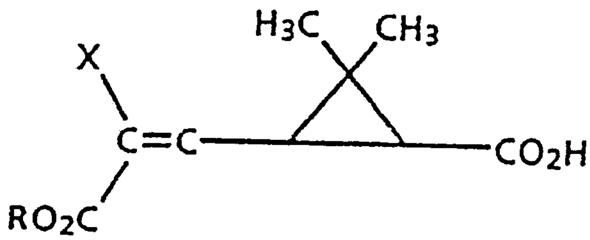

(II)

ou un dérivé fonctionnel de cet acide dans lequel X et R conservent leur signification précédente, à l'action d'un alcool de formule (III) :

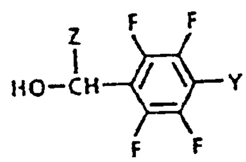

(III)

ou un dérivé fonctionnel de cet alcool dans lequel Y et Z conservent leur signification précédente pour obtenir le composé de formule (I) correspondant.

2. Procédé pour préparer les produits de formule (I) telle que définie à la revendication 1, caractérisé en ce que l'on soumet un acide de formule (IV) :

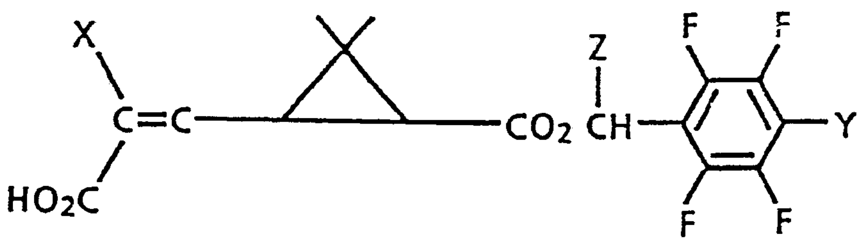

IV

dans laquelle X, Y et Z conservent leur signification précédente à l'action d'un alcool de formule V :

ROH (V)

dans laquelle R conserve sa signification précédente pour obtenir le composé de formule I correspondant.

3. Procédé selon la revendication 1, pour la préparation des produits de formule (I), telle que définie à la revendication 1, dans laquelle X, R et Z sont définis comme à la revendication 1 et Y représente un atome d'hydrogène, un radical alkyle linéaire, ramifié, saturé ou insaturé éventuellement substitué renfermant jusqu'à 8 atomes de carbone, un radical $(CH_2)_m$-O-alkyle, un radical $(CH_2)_m$-S-alkyle, un radical $(CH_2)_m$(alkyle)$_2$ dans lesquels m représente le nombre 0, 1, 3 ou 4 et renfermant jusqu'à 12 atomes de carbone ou un radical Si(alkyle)$_3$ dans lequel le radical alkyle renfermant jusqu'à 8 atomes de carbone caractérisé en ce que l'on utilise

au départ un produit de formule (II) dans laquelle X et R sont définis comme à la revendication 1 et un produit de formule (III) dans laquelle Z est défini comme à a revendication 1 et Y est défini comme ci-dessus.

4. Procédé selon la revendication 2, pour la préparation des produits de formula (I), telle que définie à la revendication 1, dans laquelle X, R et Z sont définis comme à la revendication 1 et Y représente un atome d'hydrogène, un radical alkyle linéaire, ramifié, saturé ou insaturé éventuellement substitué renfermant jusqu'à 8 atomes de carbone, un radical $(CH_2)_m$-O-alkyle, un radical $(CH_2)_m$-S-alkyle, un radical $(CH_2)_m(alkyle)_2$ dans lesquels m représente le nombre 0, 1, 3 ou 4 et renfermant jusqu'à 12 atomes de carbone ou un radical Si(alkyle)3 dans lequel le radical alkyle renfermant jusqu'à 8 atomes de carbone, caractérisé en ce que l'on utilise au départ un produit de formule (V) dans lequel R est défini comme à la revendication 1 et un produit de formule (IV) dans laquelle X et Z sont définis comme à a revendication 1 et Y est défini comme ci-dessus.

5. Procédé selon la revendication 3, caractérisé en ce que l'on utilise au départ un produit de formule (II) dans laquelle X représente un atome de fluor.

6. Procédé selon la revendication 4, caractérisé en ce que l'on utilise au départ un produit de formule (IV) dans laquelle X représente un atome de fluor.

7. Procédé selon la revendication 3, caractérisé en ce que l'on utilise au départ un produit de formule (II) dans laquelle X représente un atome d'hydrogène.

8. Procédé selon la revendication 4, caractérisé en ce que l'on utilise au départ un produit de formule (IV) dans laquelle X représente un atome d'hydrogène.

9. Procédé selon l'une quelconque des revendications 3, 5 ou 7, caractérisé en ce que l'on utilise au départ un produit de formule (II) dans laquelle R représente un radical alcoyle saturé, linéaire ou ramifié renfermant jusqu'à 4 atomes de carbone.

10. Procédé selon l'une quelconque des revendications 4, 6 ou 8, caractérisé en ce que l'on utilise au départ un produit de formule (V) dans laquelle R représente un radical alcoyle saturé, linéaire ou ramifié renfermant jusqu'à 4 atomes de carbone.

11. Procédé selon l'une quelconque des revendications 3, 5, 7 ou 9, caractérisé en ce que l'on utilise au départ un produit de formule (III) dans laquelle R représente un atome d'hydrogène.

12. Procédé selon l'une quelconque des revendications 4, 6, 8 ou 10, caractérisé en ce que l'on utilise au départ un produit de formule (IV) dans laquelle R représente un atome d'hydrogène.

13. Procédé selon l'une quelconque des revendications 3, 5, 7, 9 ou 11, caractérisé en ce que l'on utilise au départ un produit de formule (III) dans laquelle Y représente un radical $CH_3$, un radical $-(CH_2)_m-O-CH_3$ dans lequel m représente le nombre 0 ou 1 ou un radical $-CH_2-CH=CH_2$.

14. Procédé selon l'une quelconque des revendications 4, 6, 8, 10 ou 12, caractérisé en ce que l'on utilise au départ un produit de formule (IV) dans laquelle Y représente un radical $CH_3$, un radical $-(CH_2)_m-O-CH_3$ dans lequel m représente le nombre 0 ou 1 ou un radical $-CH_2-CH=CH_2$.

15. Procédé selon la revendication 1, caractérisé en ce que l'on choisit au départ les composés de formule (II) et (III) de manière telle que l'on prépare :

- le 1R/1alpha, 3alpha (Z)/ 2,2-diméthyl 3-(3-méthoxy-3-oxo-1-propényl) cyclopropane carboxylate de (4-méthoxy 2,3,5,6-tétrafluorophényl) méthyle ;
- le 1R/1alpha,3alpha (E)/ 2,2-diméthyl 3-(2-fluoro-3-éthoxy-3-oxo-1-propényl) cyclopropane carboxylate de (4-méthyl-2,3,5,6-tétrafluorophényl) méthyle) ;
- le 1R/1alpha,3alpha (E)/ 2,2-diméthyl 3-[3-(1,1-diméthyl éthoxy) 3-oxo-2-fluoro-1-propényl] cyclopropane carboxylate de (4-méthyl-2,3,5,6-tétrafluorophényl) méthyle ;
- le [1R,(1alpha,3alpha)(E)]-2,2-diméthyl 3-(3-éthoxy 2-fluoro-3-oxo 1-propényl) cyclopropane carboxylate de [4-(2-propényl) 2,3,5,6-tétrafluoro phényl] méthyle ;
- le 1R/1alpha,3alpha (E)/ 2,2-diméthyl 3-/2-fluoro 3-(1,1-diméthyl éthoxy-3-oxo-1-propényl/ cyclopropane carboxylate de (4-méthoxy 2,3,5,6-tétrafluorophényl) méthyle ;
- le 1R/1alpha,3alpha (E)/ 2,2-diméthyl 3-(2-fluoro-3-éthoxy-3-oxo-1-propényl) cyclopropane carboxylate de (4-méthoxy-2,3,5,6-tétrafluorophényl) méthyle ;
- le [1R[1alpha,3alpha (E)]] 2,2-diméthyl 3-(2-fluoro-3-éthoxy-3-oxo-1-propényl) cyclopropane carboxylate de [(4-méthoxyméthyl-2,3,5,6-tétrafluoro) phényl] méthyle ;
- le [1R[1alpha,3alpha (E)]] 2,2-diméthyl 3-[3-(1,1-diméthyléthoxy 2-fluoro-3-oxo-1-propényl] cyclopropane carboxylate de [(4-méthoxyméthyl-2,3,5,6-tétrafluoro) phényl] méthyle ;
- le [1R[1alpha,3alpha (E)] 2,2-diméthyl 3-[2-fluoro-3-méthoxy-3-oxo-1-propényl) cyclopropane carboxylate de [(4-méthoxyméthyl-2,3,5,6-tétrafluoro) phényl] méthyle ;

16. Procédé selon la revendication 2, caractérisé en ce que l'on choisit au départ les composés de formule (IV) et (V) de manière telle que l'on prépare :

- le 1R/1alpha, 3alpha (Z)/ 2,2-diméthyl 3-(3-méthoxy-3-oxo-1-propényl) cyclopropane carboxylate de (4-méthoxy 2,3,5,6-tétrafluorophényl) méthyle ;

– le 1R/1alpha,3alpha (E)/ 2,2-diméthyl 3-(2-fluoro-3-éthoxy-3-oxo-1-propényl) cyclopropane carboxylate de (4-méthyl-2,3,5,6-tétrafluorophényl) méthyle) ;

– le 1R/1alpha,3alpha (E) 2,2-diméthyl 3-[3-(1,1-diméthyl éthoxy) 3-oxo-2-fluoro-1-propényl] cyclopropane carboxylate de (4-méthyl-2,3,5,6-tétrafluorophényl) méthyle ;

– le [1R,(1alpha,3alpha)(E)]-2,2-diméthyl 3-(3-éthoxy 2-fluoro-3-oxo 1-propényl) cyclopropane carboxylate de [4-(2-propényl) 2,3,5,6-tétrafluorophényl] méthyle ;

– le 1R/1alpha,3alpha (E)/ 2,2-diméthyl 3-/2-fluoro 3-(1,1-diméthyl éthoxy-3-oxo-1-propényl/ cyclopropane carboxylate de (4-méthoxy 2,3,5,6-tétrafluorophényl) méthyle ;

– le 1R/1alpha,3alpha (E)/ 2,2-diméthyl 3-(2-fluoro-3-éthoxy-3-oxo-1-propényl) cyclopropane carboxylate de (4-méthoxy-2,3,5,6-tétrafluorophényl) méthyle ;

– le [1R[1alpha,3alpha (E)]] 2,2-diméthyl 3-(2-fluoro-3-éthoxy-3-oxo-1-propényl) cyclopropane carboxylate de [(4-méthoxyméthyl-2,3,5,6-tétrafluoro) phényl] méthyle ;

– le [1R[1alpha,3alpha (E)]] 2,2-diméthyl 3-[3-(1,1-diméthyléthoxy 2-fluoro-3-oxo-1-propényl] cyclopropane carboxylate de [(4-méthoxyméthyl-2,3,5,6-tétrafluoro) phényl] méthyle ;

– le [1R[1alpha,3alpha (E)] 2,2-diméthyl 3-[2-fluoro-3-méthoxy-3-oxo-1-propènyl) cyclopropane carboxylate de [(4-méthoxyméthyl-2,3,5,6-tétrafluoro) phényl] méthyle.

17. Les compositions destinées à la lutte contre les parasites des végétaux, les parasites des locaux et les parasites des animaux à sang chaud, caractérisées en ce qu'elles renferment comme principe actif au moins un composé défini à l'une quelconque des revendications 1 à 14.

18. Les compositions insecticides, caractérisées en ce qu'elles renferment comme principe actif au moins un composé défini à l'une quelconque des revendications 1 à 14.

19. Les compositions acaricides, caractérisées en ce qu'elles renferment comme principe actif au moins un composé défini à l'une quelconque des revendications 1 à 14.

20. Compositions douées d'activité insecticide, acaricide ou nématicide, caractérisées en ce qu'elles contiennent comme matière active, d'une part, un au moins des composés de formule générale I et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools alpha-cyano 3-phénoxy benzyliques des acides 2,2-diméthyl 3-(2,2-dichlorovinyl) cyclopropane-1-carboxyliques, par les esters d'alcools alpha-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimidométhylique, d'alcool 5-benzyl 3-furyl méthylique, d'alcool 3-phénoxy benzylique et d'alcool alpha-cyano 3-phénoxy benzyliques des acides 2,2-diméthyl 3-(-1,2,2,2-tétrahaloéthyl) cyclopropane-1-carboxyliques, dans lesquels "halo" représente un atome de fluor, de chlore ou de brome, étant entendu que les composés I peuvent exister sous toutes leurs formes stéréoisomères possibles de même que les copules acides et alcools des esters pyréthrinoïdes ci-dessus.

21. Compositions selon l'une quelconque des revendications 17 à 20, caractérisées en ce qu'elles renferment à titre de principe actif, l'un au moins des composés de formule (I) telle que définie à la revendication 3.

22. Compositions selon l'une quelconque des revendications 17 à 20, caractérisées en ce qu'elles renferment à titre de principe actif, l'un au moins des composés de formule (I) telle que définie à la revendication 4.

## Claims

**Claims for the contracting states : AT, BE, CH, DE, FR, GB, IT, LI, LU, NL, SE.**

1. The compounds of formula I

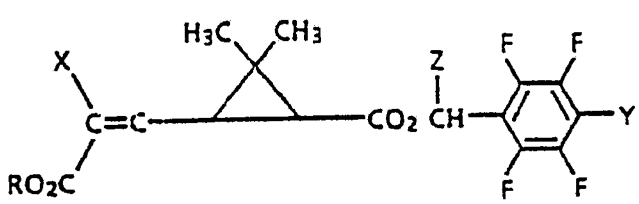

I

in which :

– X represents a hydrogen, fluorine, chlorine or bromine atom,

– R represents an optionally substituted saturated or unsaturated linear, branched or cyclic alkyl radical containing up to 8 carbon atoms, an optionally substituted aryl radical containing up to 14 carbon atoms or an optionally substituted heterocyclic radical,

– Z represents a hydrogen radical, a $CH_3$, C=N or C=CH radical, and

– Y represents ahydrogen atom, a hydroxy radical, an optionally substituted, saturated or unsaturated, linear or branched alkyl radical containing up to 8 carbon atoms, a nitrile radical, a $-(CH_2)_m$-O– alkyl radical, a $-(CH_2)_m$-S-alkyl radical, a $-(CH_2)_m$-N(alkyl)$_2$ radical, in which m represents the number 0, 1, 2, 3 or 4 and containing up to 12 carbon atoms, a Si(alkyl)$_3$ radical, alkyl represents an optionally substituted, saturated or unsaturated, linear or branched alkyl radical containing up to 8 carbon atoms, an –O-aryl or $-(CH_2)_m$-aryl radical, aryl representing an aryl radical containing up to 14 carbon atoms in all possible stereoisomer forms, as well as mixtures of these stereoisomers.

2. The compounds of formula I as defined in claim 1 inwhich X represents a fluorine atom.

3. The compounds of formula I as defined in claim 1 in which X represents a hydrogen atom.

4. The compounds of formula I as defined in any one of claims 1 to 3 in which R represents a saturated, linear or branched alkyl radical containing up to 4 carbon atoms.

5. The compounds of formula I as defined in any one of claims 1 to 4 in which Z represents a hydrogen atom.

6. The compounds of formula I as defined in any one of claims 1 to 5 in which Y represents a $CH_3$ radical, a $-(CH_2)_m$-O-$CH_3$ radical in which m represents the number 0 or 1 or a $-CH_2$-CH=$CH_2$ radical.

7. The compounds of formula I as defined in any one of claims 1 to 6 the names of which follow :

– 1R-[1alpha,3alpha (Z)]-2,2-dimethyl-3-(2-methoxy-3-oxo-1-propenyl)-cyclopropane carboxylate of (4-methoxy-2,3,5,6-tetrafluorophenyl)-methyl ;

– 1R-[1alpha,3alpha (E)]-2,2-dimethyl-3-(2-fluoro-3-ethoxy-3-oxo-1-propenyl)-cyclopropane carboxylate of (4-methyl-2,3,5,6-tetrafluorophenyl)-methyl ;

– 1R-[1alpha,3alpha (E)]-2,2-dimethyl-3-(3-(1,1-dimethylethoxy)-3-oxo-2-fluoro-1-propenyl)-cyclopropane carboxylate of (4-methyl-2,3,5,6-tetrafluorophenyl)-methyl ;

– [1R-(1alpha,3alpha) (E)]-2,2-dimethyl-3-(3-ethoxy-2-fluoro-3-oxo-1-propenyl)-cyclopropane carboxylate of [4-(2-propenyl)-2,3,5,6-tetrafluorophenyl]-methyl ;

– 1R-[1alpha,3alpha (E)]-2,2-dimethyl-3-[2-fluoro-3-(1,1-dimethyl)-ethoxy-3-oxo-1-propenyl]-cyclopropane carboxylate of (4-methoxy-2,3,5,6-tetrafluorophenyl)-methyl ;

– 1R-[1alpha,3alpha (E)]-2,2-dimethyl-3-(2-fluoro-3-ethoxy-3-oxo-1-propenyl)-cyclopropane carboxylate of (4-methoxy-2,3,5,6-tetrafluorophenyl)-methyl ;

– [1R-[1alpha,3alpha (E)]]-2,2-dimethyl-3-(2-fluoro-3-ethoxy-3-oxo-1-propenyl)-cyclopropane carboxylate of [4-methoxymethyl-2,3,5,6-tetrafluoro)-phenyl]-methyl ;

– [1R-[1alpha,3alpha (E)]]-2,2-dimethyl-3-[3-(1,1-dimethylethoxy)-2-fluoro-3-oxo-1-propenyl]-cyclopropane carboxylate of [(4-methoxymethyl-2,3,5,6-tetrafluoro)-phenyl]-methyl ;

– [1R-[1alpha,3alpha (E)]]-2,2-dimethyl-3-(2-fluoro-3-methoxy-3-oxo-1-propenyl)-cyclopropane carboxylate of [(4-methoxymethyl-2,3,5,6-tetrafluoro)-phenyl]-methyl.

8. Use of compounds of formula I as defined in any one of claims 1 to 7, in combating the parasites of vegetation and the parasites of premises.

9. The compositions intended for combating the parasites of vegetation, the parasites of premises and the parasites of warm-blooded animals, characterized in that they contain as active ingredient at least one compound defined in any one of claims 1 to 7.

10. The insecticide compositions, characterized in that they contain as active ingredient at least one compound defined in any one of claims 1 to 7.

11. The acaricide compositions, characterized in that they contain as active ingredient at least one compound defined in any one of claims 1 to 7.

12. Combinations endowed with insecticide, acaricide or nematicide activity, characterised in that they contain as active material,on the one hand at least one of the compounds of general formula (I) and on the other hand, at least one of the pyrethrinoid esters chosen from the group constituted by the esters of allethrone, of 3,4,5,6-tetrahydrophthalimidomethyl alcohol, of 5-benzyl-3-furyl methyl alcohol, of 3-phenoxybenzyl alcohol and of alpha-cyano-3-phenoxybenzyl alcohols with chrysanthemic acids, by the esters of 5-benzyl-3-furyl methyl alcohol with 2,2-dimethyl-3-(2-oxo-3-tetrahydrothiophenylidenemethyl)-cyclopropane-1-carboxylic acids, by the esters of 3-phenoxybenzyl alcohol and of alpha-cyano-3-phenoxybenzyl alcohols with 2,2-dimethyl-3-(2,2-dichlorovinyl)-cyclopropane-1-carboxylic acids, by the esters of alpha-cyano-3-phenoxy-benzyl alcohols with 2,2-dimethyl-3-(2,2-dibromovinyl)-cyclopropane-1-carboxylic acids, by the esters of 3-phenoxybenzyl alcohols with 2-parachlorophenyl-2-isopropyl acetic acids, by the esters of allethrolone, of 3,4,5,6-tetrahydrophthalimidomethyl alcohol, of 5-benzyl-3-furyl methyl alcohol, of 3-phenoxybenzyl alcohol, and of alpha-cyano-3-phenoxybenzyl alcohol with 2,2-dimethyl-3-(1,2,2,2-tetrahaloethyl)-cyclopropane-1-carboxylic acids, in which "halo" represents a fluorine,chlorine or bromine atom, it being understood that the compounds I can exisit in all their possible strereoisomer forms as well as the acid and alcohol copulas of the above pyrethrinoid esters.

13. Preparation process for compounds of formula I as defined in any one of claims 1 to 7, characterized in that an acid of formula II :

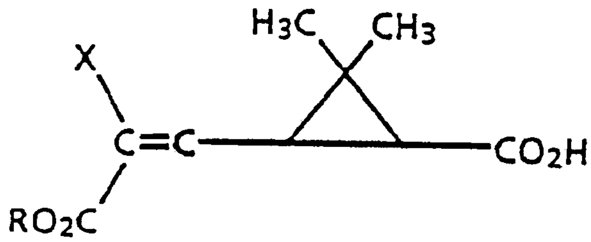

II

or a functional derivative of this acid in which X and R retain their previous meaning, is subjected to the action of an alcohol of formula III :

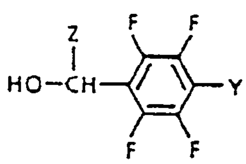

III

or a functional derivative of this alcohol in which Y and Z retain their previous meaning in order to obtain the corresponding compound of formula I.

14. Variant of the process of claim 13, characterized in that an acid of formula IV :

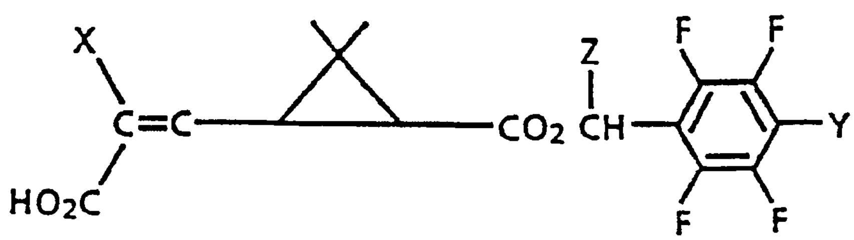

IV

in which X, Y and Z retain their previous meaning is subjected to the action of an alcohol of formula V :

ROH (V)

in which R retains its previous meaning, in order to obtain the corresponding compound of formula I.

15. As new chemical products, the compounds of formula III as defined in claim 13 in which Z does not represent a hydrogen atom.

16. As new chemical products, the compounds of formula III defined in claim 13 the names of which follow:

- alpha-methyl-4-methyl-2,3,5,6-tetrafluorobenzyl alcohol ;
- alpha-cyano-4-methyl-2,3,5,6-tetrafluorobenzyl alcohol ;
- alpha-methyl-4-cyano-2,3,5,6-tetrafluorobenzyl alcohol ;
- 4-[(1,1-dimethyl-ethyl)-dimethyl-siloxy]-2,3,5,6-tetrafluorobenzyl alcohol ;
- 4-bromo-difluoromethoxy-2,3,5,6-tetrafluorobenzyl alcohol ;
- 4-difluoromethoxy-2,3,5,6-tetrafluorobenzyl alcohol ;
- 2-[[2,3,5,6-tetrafluoro-4-(1,2-propa-dienyl)-phenyl]-methoxy] tetrahydropryran.

**Claims for the contracting state : ES**

1. Process for preparing the compounds of formula I :

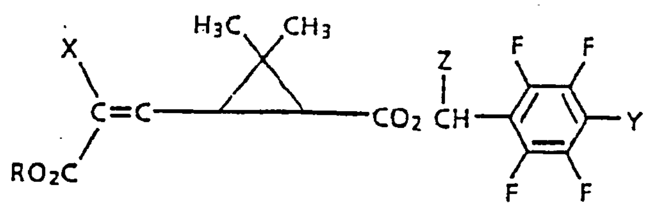

I

in which

- X represents a hydrogen, fluorine, chlorine or bromine atom,
- R represents an optionally substituted saturated or unsaturated linear, branched or cyclic alkyl radical containing up to 8 carbon atoms, an optionally substituted aryl radical containing up to 14 carbon atoms or an optionally substituted heterocyclic radical,
- Z represents a hydrogen atom, a $CH_3$, C=N or C=CH radical, and
- Y represents a hydrogen atom, a hydroxy radical, an optionally substituted, saturated or unsaturated, linear or branched alkyl radical containing up to 8 carbon atoms, a nitrile radical, a $-(CH_2)_m$-O– alkyl radical, a $-(CH_2)_m$-S– alkyl radical, a $-(CH_2)_m$-N(alkyl)$_2$ radical, in which m represents the number 0, 1, 2, 3 or 4 and containing up to 12 carbon atoms, a Si(alkyl)$_3$ radical, alkyl represents an optionally substituted, saturated or unsaturated, linear or branched alkyl radical containing up to 8 carbon atoms, an O-aryl or $-(CH_2)_m$-aryl radical, aryl representing an aryl radical containing up to 14 carbon atoms, in all possible stereoisomer forms, as well as mixtures of these stereoisomers, characterized in that an acid of formula II :

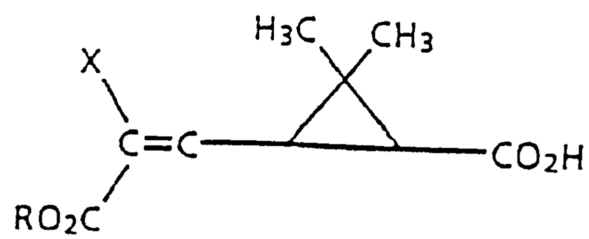

II

or a functional derivative of this acid in which X and R retain their previous meaning, is subjected to the action of an alcohol of formula III :

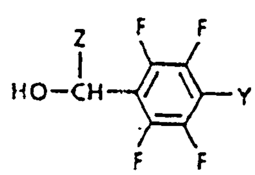

III

or a functional derivative of this alcohol in which Y and Z retain their previous meaning in order to obtain the corresponding compound of formula I.

2. Process for preparing the products of formula (I) as defined in claim 1, characterized in that an acid of formula (IV) :

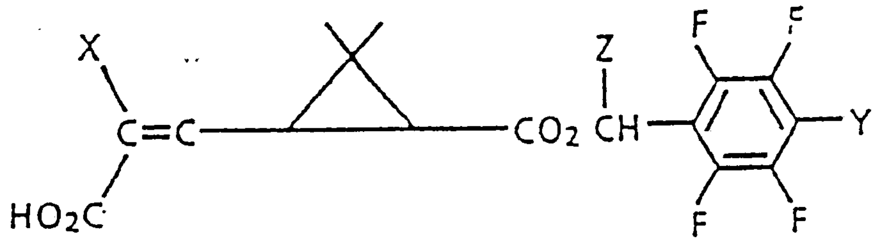

(IV)

in which X, Y and Z retain their previous meaning, is subjected to the action of an alcohol of formula V :

ROH (V)

in which R retains its previous meaning in order to obtain the corresponding compound of formula I.

3. Process according to claim 1, for the preparation of products of formula (I), as defined in claim 1, in which X, R and Z are as defined in claim 1 and Y represents a hydrogen atom, an optionally substituted, saturated or unsaturated, linear or branched alkyl radical containing up to 8 carbon atoms, a $(CH_2)_m$-O-alkyl radical, a $(CH_2)_m$-S-alkyl radical, a $(CH_2)_m(alkyl)_2$ radical in which m represents the number 0, 1, 3 or 4 and containing up to 12 carbon atoms or an $Si(alkyl)_3$ radical in which the alkyl radical contains up to 8 carbon atoms, characterized in that at the start a product of formula (II) in which X and R are defined as in claim 1 and a product of formula (III) in which Z is defined as in claim 1 and Y is as defined above, are used.

4. Process according to claim 2, for the preparation of products of formula (I), as defined in claim 1, in which X, R and Z are as defined in claim 1 and Y represents a hydrogen atom, an optionally substituted, saturated or unsaturated, linear or branched alkyl radical containing up to 8 carbon atoms, a $(CH_2)_m$-O-alkyl radical, a $(CH_2)_m$-S-alkyl radical, a $(CH_2)_m(alkyl)_2$ radical in which m represents the number 0, 1, 3 or 4 and containing up to 12 carbon atoms or a $Si(alkyl)_3$ radical in which the alkyl radical contains up to 8 carbon atoms, characterized in that at the start a product of formula (V) in which R is defined as in claim 1 and a product of formula (IV) in which X and Z are as defined in claim 1 and Y is as defined above, are used.

5. Process according to claim 3, characterized in that at the start a product of formula (II) is used in which X represents a fluorine atom.

6. Process according to claim 4, characterized in that at the start a product of formula (IV) is used in which X represents a fluorine atom.

7. Process according to claim 3, characterized in that at the start a product of formula (II) is used in which X represents a hydrogen atom.

8.Process according to claim 4, characterized in that at the start a product of formula (IV) is used in which X represents a hydrogen atom.

9. Process according to any one of claims 3, 5 or 7, characterized in that at the start a product of formula (II) is used in which R represents a saturated, linear or branched alkyl radical containing up to 4 carbon atoms.

10. Process according to any one of claims 4, 6 or 8, characterized in that at the start a product of formula (V) is used in which R represents a saturated, linear or branched alkyl radical containing up to 4 carbon atoms.

11. Process according to any one of claims 3, 5, 7 or 9, characterized in that at the start a product of formula (III) is used in which R represents a hydrogen atom.

12. Process according to any one of claims 4, 6, 8 or 10, characterized in that in that at the start a product of formula (IV) is used in which R represents a hydrogen atom.

13. Process according to any one of claims 3, 5, 7, 9 or 11, characterized in that at the start a product of formula (III) is used in which Y represents a $CH_3$ radical, a $-(CH_2)_m-O-CH_3$ radical in which m represents the number 0 or 1 or a $-CH_2-CH=CH_2$ radical.

14. Process according to any one of claims 4, 6, 8, 10 or 12, characterized in that at the start a product of formula (IV) is used in which Y represents a $CH_3$ radical, a $-(CH_2)_m-O-CH_3$ radical in which m represents the number 0 or 1 or a $-CH_2-CH=CH_2$ radical.

**Claims for contracting state : GR**

1. Process for preparing the compounds of formula I :

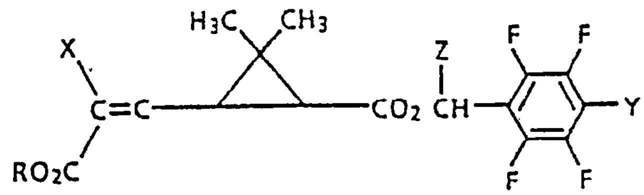

I

in which

– X represents a hydrogen, fluorine, chlorine or bromine atom,

– R represents an optionally substituted saturated or unsaturated linear, branched or cyclic alkyl radical containing up to 8 carbon atoms, an optionally substituted aryl radical containing up to 14 carbon atoms or an optionally substituted heterocyclic radical,

– Z represents a hydrogen radical, a $CH_3$, C=N or C=CH radical, and

– Y represents a hydrogen atom, a hydroxy radical, an optionally substituted, saturated or unsaturated, linear or branched alkyl radical containing up to 8 carbon atoms, a nitrile radical, a $-(CH_2)_m$-O-alkyl radical, a $-(CH_2)_m$-S-alkyl radical, a $-(CH_2)_m-N(alkyl)_2$ radical, in which m represents the number 0, 1, 2, 3 or 4 and containing up to 12 carbon atoms, an $Si(alkyl)_3$ radical, alkyl represents an optionally substituted, saturated or unsaturated, linear or branched alkyl radical containing up to 8 carbon atoms, an –O-aryl or $-(CH_2)_m$-aryl radical, aryl representing an aryl radical containing up to 14 carbon atoms in all possible stereoisomer forms, as well as mixtures of these stereoisomers, characterized in that an acid of formula II :

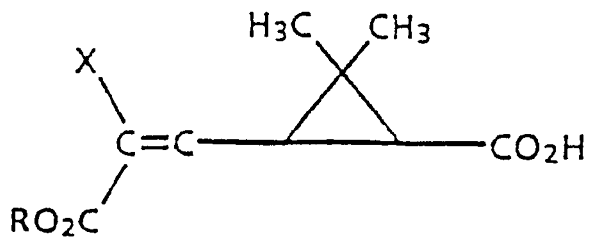

II

or a functional derivative of this acid in which X and R retain their previous meaning, is subjected to the action of an alcohol of formula III :

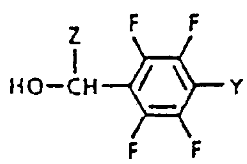

III

or a functional derivative of this alcohol in which Y and Z retain their previous meaning in order to obtain the corresponding compound of formula I.

2. Process for preparing the products of formula (I) as defined in claim 1, characterized in that an acid of formula (IV) :

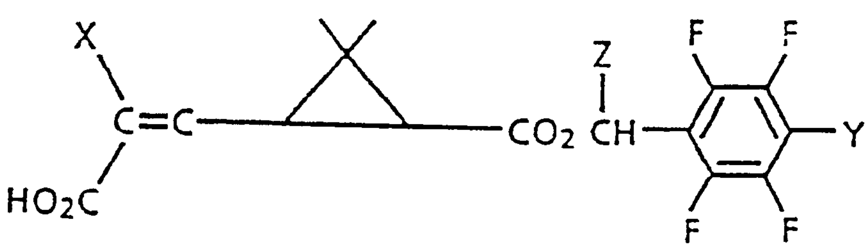

(IV)

in which X, Y and Z retain their previous meaning is subjected to the action of an alcohol of formula V :

ROH (V)

in which R retains its previous meaning in order to obtain the corresponding compound of formula I.

3. *Process according to claim 1, for the preparation of products of formula (I), as defined in claim 1, in which* X, R and Z are as defined in claim 1 and Y represents a hydrogen atom, an optionally substituted, saturated or unsaturated, linear or branched alkyl radical containing up to 8 carbon atoms, a $(CH_2)_m$-O-alkyl radical, a $(CH_2)_m$-S-alkyl radical, a $(CH_2)_m(alkyl)_2$ radical in which m represents the number 0, 1, 3 or 4 and containing up to 12 carbon atoms or an $Si(alkyl)_3$ radical in which the alkyl radical contains up to 8 carbon atoms, characterized in that at the start a product of formula (II) in which X and R are defined as in claim 1 and a product of formula (III) in which Z is defined as in claim 1 and Y is as defined above, are used.

4. Process according to claim 2, for the preparation of products of formula (I), as defined in claim 1, in which X, R and Z are as defined in claim 1 and Y represents a hydrogen atom, an optionally substituted, saturated or unsaturated, linear or branched alkyl radical containing up to 8 carbon atoms, a $(CH_2)_m$-O-alkyl radical, a $(CH_2)_m$-S-alkyl radical, a $(CH_2)_m$(alkyl)2 radical in which m represents the number 0, 1, 3 or 4 and containing up to 12 carbon atoms or an $Si(alkyl)_3$ radical in which the alkyl radical contains up to 8 carbon atoms, characterized in that in that at the start a product of formula (V) in which R is defined as in claim 1 and a product of formula (IV) in which X and Z are as defined in claim 1 and Y is as defined above, are used.

5. Process according to claim 3, characterized in that at the start a product of formula (II) is used in which X represents a fluorine atom.

6. Process according to claim 4, characterized in that in that at the start a product of formula (IV) is used in which X represents a fluorine atom.

7. Process according to claim 3, characterized in that at the start a product of formula (II) is used in which X represents a hydrogen atom.

8.Process according to claim 4, characterized in that at the start a product of formula (IV) is used in which X represents a hydrogen atom.

9. Process according to any one of claims 3, 5 or 7, characterized in that at the start a product of formula (II) is used in which R represents a saturated, linear or branched alkyl radical containing up to 4 carbon atoms.

10. Process according to any one of claims 4, 6 or 8, characterized in that at the start a product of formula (V) is used in which R represents a saturated, linear or branched alkyl radical containing up to 4 carbon atoms.

11. Process according to any one of claims 3, 5, 7 or 9, characterized in that at the start a product of formula (III) is used in which R represents a hydrogen atom.

12. Process according to any one of claims 4, 6, 8 or 10, characterized in that at the start a product of formula (IV) is used in which R represents a hydrogen atom.

13. Process according to any one of claims 3, 5, 7, 9 or 11, characterized in that at the start a product of formula (III) is used in which Y represents a CH3 radical, a $-(CH_2)_m-O-CH_3$ radical in which m represents the number 0 or 1 or a $-CH_2-CH=CH_2$ radical.

14. Process according to any one of claims 4, 6, 8, 10 or 12, characterized in that at the start a product of formula (IV) is used in which Y represents a $CH_3$ radical, a $-(CH_2)_m-O-CH_3$ radical in which m represents the number 0 or 1 or a $-CH2-CH=CH_2$ radical.

15. Process according to claim 1, characterized in that at the start the compounds of formula (II) and (III) are chosen in such a way so as to prepare the following :

- 1R-[1alpha,3alpha (Z)]-2,2-dimethyl-3-(3-methoxy-3-oxo-1-propenyl)-cyclopropane carboxylate of (4-methoxy-2,3,5,6-tetrafluorophenyl)-methyl ;
- 1R-[1alpha,3alpha (E)]-2,2-dimethyl-3-(2-fluoro-3-ethoxy-3-oxo-1-propenyl)-cyclopropane carboxylate of (4-methyl-2,3,5,6-tetrafluorophenyl)-methyl ;
- 1R-[1alpha,3alpha (E)]-2,2-dimethyl-3-[3-(1,1-dimethyl-ethoxy)-3-oxo-2-fluoro-1-propenyl]-cyclopropane carboxylate of (4-methyl-2,3,5,6-tetrafluorophenyl)-methyl ;
- [1R-(1alpha,3alpha) (E)]-2,2-dimethyl-3-(3-ethoxy-2-fluoro-3-oxo-1-propenyl)-cyclopropane carboxylate of [4-(2-propenyl)-2,3,5,6-tetrafluorophenyl]-methyl ;
- 1R-[1alpha,3alpha (E)]-2,2-dimethyl-3-2-fluoro-3-(1,1-dimethyl-ethoxy-3-oxo-1-propenyl)-cyclopropane carboxylate of (4-methoxy-2,3,5,6-tetrafluorophenyl)-methyl ;
- 1R-[1alpha,3alpha (E)]-2,2-dimethyl-3-(2-fluoro-3-ethoxy-3-oxo-1-propenyl)-cyclopropane carboxylate of (4-methoxy-2,3,5,6-tetrafluorophenyl)-methyl ;
- [1R-[1alpha,3alpha (E)]]-2,2-dimethyl-3-(2-fluoro-3-ethoxy-3-oxo-1-propenyl)-cyclopropane carboxylate of [(4-methoxymethyl2,3,5,6-tetrafluoro)-phenyl]-methyl ;
- [1R-[1alpha,3alpha (E)]]-2,2-dimethyl-3-[3-(1,1-dimethylethoxy-2-fluoro-3-oxo-1-propenyl)-cyclopropane carboxylate of [4-methoxymethyl-2,3,5,6-tetrafluoro)-phenyl]-methyl ;
- [1R-[1alpha,3alpha (E)]]-2,2-dimethyl-3-(2-fluoro-3-methoxy-3-oxo-1-propenyl)-cyclopropane carboxylate of [(4-methoxymethyl-2,3,5,6-tetrafluoro)-phenyl]-methyl.

16. Process according to claim 2, characterized in that the compounds of formula (IV) and (V) are chosen

in such a way so as to prepare the following :

– 1R-[1alpha,3alpha (Z)]-2,2-dimethyl-3-(3-methoxy-3-oxo-1propenyl)-cyclopropane carboxylate of (4-methoxy-2,3,5,6-tetrafluorophenyl)-methyl ;

– 1R-[1alpha,3alpha (E)]-2,2-dimethyl-3-(2-fluoro-3-ethoxy-3-oxo-1-propenyl)-cyclopropane carboxylate of (4-methyl-2,3,5,6-tetrafluorophenyl)-methyl ;

– 1R-[1alpha,3alpha (E)]-2,2-dimethyl-3-[3-(1,1-dimethyl-ethoxy)-3-oxo-2-fluoro-1-propenyl]-cyclopropane carboxylate of (4-methyl-2,3,5,6-tetrafluorophenyl)-methyl ;

– [1R-(1alpha,3alpha) (E)]-2,2-dimethyl-3-(3-ethoxy-2-fluoro3-oxo-1-propenyl)-cyclopropane carboxylate of [4-(2-propenyl)-2,3,5,6-tetrafluorophenyl]-methyl ;

– 1R-[1alpha,3alpha (E)]-2,2-dimethyl-3-[2-fluoro-3-(1,1-dimethyl-ethoxy-3-oxo-1-propenyl]-cyclopropane carboxylate of (4-methoxy-2,3,5,6-tetrafluorophenyl)-methyl ;

– 1R-[1alpha,3alpha (E)]-2,2-dimethyl-3-(2-fluoro-3-ethoxy-3-oxo-1-propenyl)-cyclopropane carboxylate of (4-methoxy-2,3,5,6-tetrafluorophenyl)-methyl ;

– [1R-[1alpha,3alpha (E)]]-2,2-dimethyl-3-(2-fluoro-3-ethoxy-3-oxo-1-propenyl)-cyclopropane carboxylate of [(4-methoxymethyl-2,3,5,6-tetrafluoro)-phenyl]-methyl ;

– [1R-[1alpha,3alpha (E)]]-2,2-dimethyl-3-[3-(1,1-dimethylethoxy-2-fluoro-3-oxo-1-propenyl)-cyclopropane carboxylate of [(4-methoxymethyl-2,3,5,6-tetrafluoro)-phenyl]-methyl ;

– [1R-[1alpha,3alpha (E)]-2,2-dimethyl-3-(2-fluoro-3-methoxy-3-oxo-1-propenyl)-cyclopropane carboxylate of [4-methoxymethyl-2,3,5,6-tetrafluoro)-phenyl]-methyl.

17. The compositions intended for combating the parasites of vegetation, the parasites of premises and the parasites of warm-blooded animals, characterized in that they contain as active ingredient at least one compound defined in any one of claims 1 to 14.

18. The insecticide compositions, characterized in that they contain as active ingredient at least one compound defined in any one of claims 1 to 14.

19. The acaricide compositions, characterized in that they contain as active ingredient at least one compound defined in any one of claims 1 to 14.

20. Compositions endowed with insecticide, acaricide or nematicide activity, characterised in that they contain as active material,on the one hand at least one of the compounds of general formula (I) and on the other hand, at least one of the pyrethrinoid esters chosen from the group constituted by the esters of allethrone, of 3,4,5,6-tetrahydrophthalimidomethyl alcohol, of 5-benzyl-3-furyl methyl alcohol, of 3-phenoxybenzyl alcohol and of alpha-cyano-3-phenoxybenzyl alcohols with chrysanthemic acids, by the esters of 5-benzyl-3-furyl methyl alcohol with 2,2-dimethyl-3-(2-oxo-3-tetrahydrothiophenylidenemethyl)-cyclopropane-1-carboxylic acids, by the esters of 3-phenoxybenzyl alcohol and of alpha-cyano-3-phenoxybenzyl alcohols with 2,2-dimethyl-3-(2,2-dichlorovinyl)-cyclopropane-1-carboxylic acids, by the esters of alpha-cyano-3-phenoxy-benzyl alcohols with 2,2-dimethyl-3-(2,2-dibromovinyl)-cyclopropane-1-carboxylic acids, by the esters of 3-phenoxybenzyl alcohols with 2-parachlorophenyl-2-isopropyl acetic acids, by the esters of allethrolone, of 3,4,5,6-tetrahydrophthalimidomethyl alcohol, of 5-benzyl-3-furyl methyl alcohol, of 3-phenoxybenzyl alcohol, and of alpha-cyano-3-phenoxybenzyl alcohol with 2,2-dimethyl-3-(1,2,2,2-tetrahaloethyl)-cyclopropane-1-carboxylic acids, in which "halo" represents a fluorine,chlorine or bromine atom, it being understood that the compounds I can exisit in all their possible stereoisomer forms as well as the acid and alcohol copulas of the above pyrethrinoid esters.

21. Compositions according to any one of claims 17 to 20, characterized in that they contain as active ingredient, at least one of the compounds of formula (I) as defined in claim 3.

22. Compositions according to any one of claims 17 to 20, characterized in that they contain as active ingredient, at least one of the compounds of formula (I) as defined in claim 4.

**Ansprüche**

**Patentansprüche für die Vertragsstaten : AT, BE, CH, DE, FR, GB, IT, LI, LU, NL, SE**

1. Verbindungen der Formel I

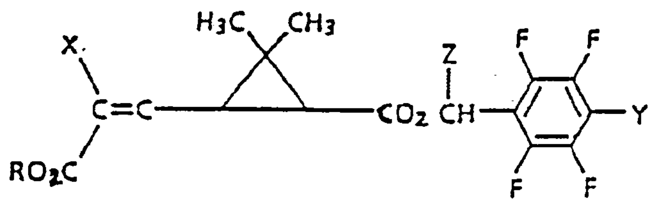

(I)

worin :

– X ein Wasserstoff-, Fluor-, Chlor- oder Bromatom bedeutet,

– R einen linearen, verzweigten oder cyclischen, gesättigten oder ungesättigten, gegebenenfalls substituierten Alkylrest mit bis zu 8 Kohlenstoffatomen, einen gegebenenfalls substituierten Arylrest mit bis zu 14 Kohlenstoffatomen oder einen gegebenenfalls substituierten, heterocyclischen Rest darstellt,

– Z ein Wasserstoffatom, einen Rest $CH_3$, C≡N oder C≡CH bedeutet und

– Y ein Wasserstoffatom, einen Hydroxyrest, einen linearen, verzweigten, gesättigten oder ungesättigten, gegebenenfalls substituierten Alkylrest mit bis zu 8 Kohlenstoffatomen, einen Nitrilrest, einen Rest $-(CH_2)_m$-O-alkyl, $-(CH_2)_m$-S-alkyl, $-(CH_2)_m$-N-(alkyl)$_2$, worin m die Zahl 0, 1, 2, 3 oder 4 ist und mit bis zu 12 Kohlenstoffatomen, einen Si(alkyl)$_3$-Rest, wobei Alkyl einen linearen oder verzweigten, gesättigten oder ungesättigten, gegebenenfalls substituierten Alkylrest mit bis zu 8 Kohlenstoffatomen darstellt, einen Rest –O-Aryl– oder $-(CH_2)_m$-Aryl bedeutet, wobei Aryl einen Arylrest mit bis zu 14 Kohlenstoffatomen darstellt, unter allen möglichen stereoisomeren Formen sowie den Gemischen dieser Stereoisomeren.

2. Verbindungen der Formel I, wie in Anspruch 1 definiert, worin X ein Fluoratom bedeutet.

3. Verbindungen der Formel I, wie in Anspruch 1 definiert, worin X ein Wasserstoffatom bedeutet.

4. Verbindungen der Formel I, wie in einem der Ansprüche 1 bis 3 defiliert, worin R einen gesättigten, linearen oder verzweigten Alkylrest mit bis zu 4 Kohlenstoffatomen bedeutet.

5. Verbindungen der Formel I, wie in einem der Ansprüche 1 bis 4 definiert, worin Z ein Wasserstoffatom bedeutet.

6. Verbindungen der Formel I, wie in einem der Ansprüche 1 bis 5 definiert, worin Y einen Rest $CH_3$, einen Rest $-(CH_2)_m-O-CH_3$, worin m die Zahl 0 oder 1 bedeutet, oder einen Rest $-CH_2-CH=CH_2$ darstellt.

7. Verbindungen der Formel I, wie in einem der Ansprüche 1 bis 6 defiliert, mit folgenden Namen :

1R-[1α,3α-(Z)]-2,2-Dimethyl-3-(3-methoxy-3-oxo-1-propenyl)-cyclopropan-carbonsäure-(4-methoxy-2,3,5,6-tetrafluorphenyl)-methylester ;

1R-[1α,3α-(E)]-2,2-Dimethyl-3-(2-fluor-3-ethoxy-3-oxo-1-propenyl)-cyclopropan-carbonsäure-(4-methyl-2,3,5,6-tetrafluorphenyl)-methylester ;

1R-[1α,3α-(E)]-2,2-Dimethyl-3-[3-(1,1-dimethylethoxy)-3-oxo-2-fluor-1-propenyl]-cyclopropan-carbonsäure-(4-methyl-2,3,5,6-tetrafluorphenyl)-methylester ;

[1R-(1α,3α)-(E)]-2,2-Dimethyl-3-(3-ethoxy-2-fluor-3-oxo-1-propenyl)-cyclopropan-carbonsäure-[4-(2-propenyl)-2,3,5,6-tetrafluorphenyl]-methylester ;

1R-[1α,3α-(E)]-2,2-Dimethyl-3-[2-fluor-3-(1,1-dimethylethoxy-3-oxo-1-propenyl]-cyclopropan-carbonsäure-(4-methoxy-2,3,5,6-tetrafluorphenyl)-methylester ;

1R-[1α,3α-(E)]-2,2-Dimethyl-3-(2-fluor-3-ethoxy-3-oxo-1-propenyl)-cyclopropan-carbonsäure-(4-methoxy-2,3,5,6-tetrafluorphenyl)-methylester ;

[1R-[1α,3α-(E)]]-2,2-Dimethyl-3-(2-fluor-3-ethoxy-3-oxo-1-propenyl)-cyclopropan-carbonsäure-[(4-methoxymethyl-2,3,5,6-tetrafluor)-phenyl]-methylester ;

[1R-[1α,3α-(E)]]-2,2-Dimethyl-3-[3-(1,1-dimethylethoxy-2-fluor-3-oxo-1-propenyl]-cyclopropan-carbonsäure-[(4-methoxymethyl-2,3,5,6-tetrafluor)-phenyl]-methylester ;

[1R-[1α,3α-(E)]]-2,2-Dimethyl-3-[2-fluor-3-methoxy-3-oxo-1-propenyl)-cyclopropan-carbonsäure-[(4-methoxymethyl-2,3,5,6-tetrafluor)-phenyl]-methylester.

8. Verwendung der Verbindungen der Formel I, wie in einem der Ansprüche 1 bis 7 definiert, zur Bekämpfung von pflanzlichen Parasiten und Parasiten in Räumlichkeiten.

9. Zusammensetzungen, bestimmt zur Bekämpfung der Pflanzenparasiten, der Parasiten in Räumlichkeiten und der Parasiten von Warmblütern, dadurch gekennzeichnet, daß sie als aktives Prinzip wenigstens eine Verbindung, definiert in einem der Ansprüche 1 bis 7, enthalten.

10. Insektizide Zusammensetzungen, dadurch gekennzeichnet, daß sie als aktives Prinzip wenigstens eine Verbindung, definiert in einem der Ansprüche 1 bis 7, enthalten.

11. Akarizide Zusammensetzungen, dadurch gekennzeichnet, daß sie als aktives Prinzip wenigstens eine Verbindung, defiliert in einem der Ansprüche 1 bis 7, enthalten.

12. Assoziationen mit insektizider, akarizider oder nematizider Aktivität, dadurch gekennzeichnet, daß sie

als aktives Material einesteils zumindest eine der Verbindungen der allgemeinen Formel (I) und anderenteils zumindest einen Pyrethrinoidester, ausgewählt aus der Gruppe, bestehend aus den Estern der Allethrolone, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der Chrysanthemumsäuren, den Estern des 5-Benzyl-3-furylmethylalkohols der 2,2-Dimethyl-3-(2-oxo-3-tetrahydrothiophenylidenmethyl)-cyclopropan-1-carbonsäuren, den Estern des 3-Phenoxybenzylalkohols und der α-Cyano-3-phenoxybenzylalkohole der 2,2-Dimethyl-3-(2,2-dichlorvinyl)-cyclopropan-1-carbonsäuren, den Estern der α-Cyano-3-phenoxybenzylalkohole der 2,2-Dimethyl-3-(2,2-dibromvinyl)-cyclopropan-1-carbonsäuren, den Estern des 3-Phenoxybenzylalkohols der 2-p-Chlorphenyl-2-isopropylessigsäuren, den Estern der Allethrolone, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der 2,2-Dimethyl-3-(1,2,2,2-tetrahaloethyl)-cyclopropan-1-carbonsäuren, enthalten, worin "halo" ein Fluor-, Chlor- oder Bromatom wiedergibt, mit der Maßgabe, daß die Verbindungen I in sämtlichen ihrer möglichen stereoisomeren Formen ebenso wie die Verbindungen der Säuren und Alkohole der vorstehenden Pyrethrinoidester vorliegen können.

13. Verfahren zur Herstellung der Verbindungen der Formel I, wie in einem der Ansprüche 1 bis 7 defiliert, dadurch gekennzeichnet, daß man eine Säure der Formel II

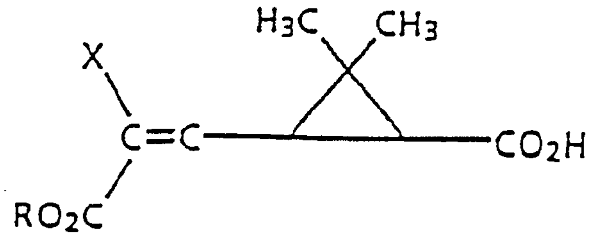

(II)

oder ein funktionelles Derivat dieser Säure, worin X und R die vorstehende Bedeutung haben, der Einwirkung eines Alkohols der Formel (III)

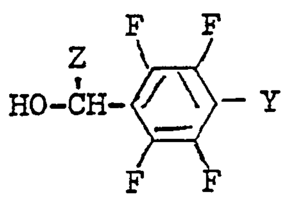

(III)

oder eines funktionellen Derivats dieses Alkohols, worin Y und Z die vorstehende Bedeutung haben, unterwirft, um die entsprechende Verbindung der Formel (I) zu erhalten.

14. Variante des Verfahrens gemäß Anspruch 13, dadurch gekennzeichnet, daß man eine Säure der Formel IV

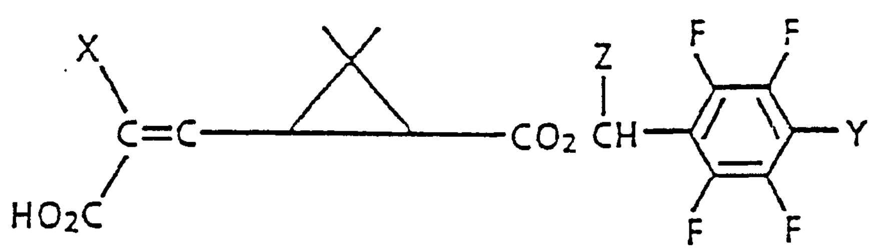

(IV).

worin X, Y und Z die vorstehende Bedeutung haben, der Einwirkung eines Alkohols der Formel V

ROH (V)

worin R die Vorstehende Bedeutung hat, unterwirft, um die entsprechende Verbindung der Formel I zu erhalten.

15. Als neue Produkte die Verbindungen der Formel III, wie sie in Anspruch 13 defiliert sind, worin Z nicht ein Wasserstoffatom bedeutet.

16. Als neue, chemische Produkte die Verbindungen der Formel III, wie sie in Anspruch 13 defiliert sind,

mit folgenden Namen :

α-Methyl-4-methyl-2,3,5,6-tetrafluorbenzylalkohol ;
α-Cyano-4-methyl-2,3,5,6-tetrafluorbenzylalkohol ;
α-Methyl-4-cyano-2,3,5,6-tetrafluorbenzylalkohol ;
4-[(1,1-Dimethylethyl)-dimethylsiloxy]-2,3,5,6-tetrafluorbenzylalkohol ;
4-Brom-difluormethoxy-2,3,5,6-tetrafluorbenzylalkohol ;
4-Difluormethoxy-2,3,5,6-tetrafluorbenzylalkohol ;
2-[[2,3,5,6-Tetrafluor-4-(1,2-propa-dienyl)-phenyl]-methoxy]-tetrahydropyran.

**Patentansprüche für den Vertragsstaat : ES**

1. Verfahren zur Herstellung der Verbindungen der Formel (I)

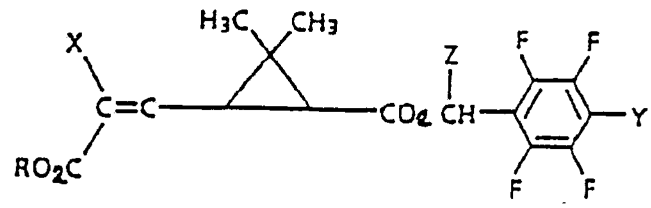

(I)

worin :

- X ein Wasserstoff-, Fluor-, Chlor- oder Bromatom bedeutet,
- R einen linearen, verzweigten oder cyclischen, gesättigten oder ungesättigten, gegebenenfalls substituierten Alkylrest mit bis zu 8 Kohlenstoffatomen, einen gegebenenfalls substituierten Arylrest mit bis zu 14 Kohlenstoffatomen oder einen gegebenenfalls substituierten, heterocyclischen Rest darstellt,
- Z ein Wasserstoffatom, einen Rest $CH_3$, C≡N oder C≡CH bedeutet und
- Y ein Wasserstoffatom, einen Hydroxyrest, einen linearen, verzweigten, gesättigten oder ungesättigten, gebenenfalls substituierten Alkylrest mit bis zu 8 Kohlenstoffatomen, einen Nitrilrest, einen Rest $-(CH_2)_m$-O-alkyl, $-(CH_2)_m$-S-alkyl, $-(CH_2)_m$-N-(alkyl)$_2$, worin m die Zahl 0, 1, 2, 3 oder 4 ist und mit bis zu 12 Kohlenstoffatomen, einen Si(alkyl)$_3$-Rest, wobei Alkyl einen linearen oder verzweigten, gesättigten oder ungesättigten, gegebenenfalls substituierten Alkylrest mit bis zu 8 Kohlenstoffatomen darstellt, einen Rest –O-Aryl– oder $-(CH_2)_m$-Aryl bedeutet, wobei Aryl einen Arylrest mit bis zu 14 Kohlenstoffatomen darstellt, unter allen möglichen stereoisomeren Formen sowie den Gemischen dieser Stereoisomeren, dadurch gekennzeichnet, daß man eine Säure der Formel (II)

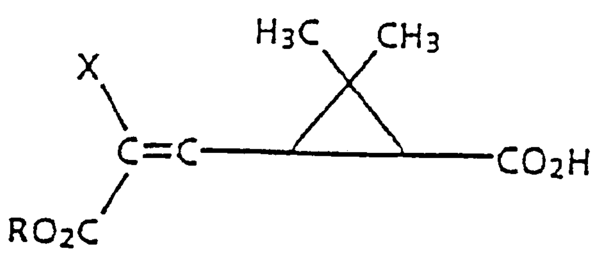

(II)

oder ein funktionelles Derivat dieser Säure, worin X und R die vorstehende Bedeutung haben, der Einwirkung eines Alkohols der Formel (III)

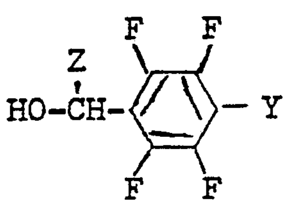

(III)

oder eines funktionellen Derivats dieses Alkohols, worin Y und Z die vorstehende Bedeutung haben, unterwirft, um die entsprechende Verbindung der Formel (I) zu erhalten.

2. Verfahren zur Herstellung der Produkte der Formel (I), wie in Anspruch 1 definiert, dadurch gekennzeichnet, daß man eine Säure der Formel (IV)

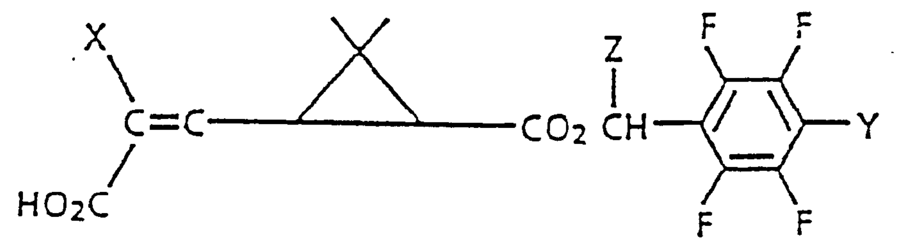

(IV)

worin X, Y und Z die vorstehende Bedeutung haben, der Einwirkung eines Alkohols der Formel V

ROH (V)

worin R die vorstehende Bedeutung hat, unterwirft, um die entsprechende Verbindung der Formel (I) zu erhalten.

3. Verfahren gemäß Anspruch 1 zur Herstellung von Produkten der Formel (I), wie in Anspruch 1 definiert, worin X, R und Z wie in Anspruch 1 defiliert sind und Y ein Wasserstoffatom, einen linearen, verzweigten, gesättigten oder ungesättigten, gegebenenfalls substituierten Alkylrest mit bis zu 18 Kohlenstoffatomen bedeutet, einen Rest $(CH_2)_m$-O-alkyl, einen Rest $(CH_2)_m$-S-alkyl, einen Rest $(CH_2)_m$-$(alkyl)_2$, worin m die Zahl 0, 1, 3 oder 4 ist und mit bis zu 12 Kohlenstoffatomen, oder einen Rest Si-$(alkyl)_3$ bedeutet, wobei der Alkylrest bis zu 8 Kohlenstoffatome enthält, dadurch gekennzeichnet, daß man als Ausgangsmaterial ein Produkt der Formel (II) verwendet, worin X und R wie in Anspruch 1 definiert sind, und ein Produkt der Formel (III) verwendet, worin Z wie in Anspruch 1 definiert ist und Y wie vorstehend definiert ist.

4. Verfahren gemäß Anspruch 2 zur Herstellung von Produkten der Formel (I), wie in Anspruch 1 definiert, worin X, R und Z wie in Anspruch 1 definiert sind und Y ein Wasserstoffatom, einen linearen, verzweigten, gesättigten oder ungesättigten, gegebenenfalls substituierten Alkylrest mit bis zu 8 Kohlenstoffatomen bedeutet, einen Rest $(CH_2)_m$-O-alkyl, einen Rest $(CH_2)_m$-S-alkyl, einen Rest $(CH_2)_m$-$(alkyl)_2$ bedeutet, worin m die Zahl 0, 1, 3 oder 4 ist und mit bis zu 12 Kohlenstoffatomen, oder einen Rest Si-$(alkyl)_3$ bedeutet, wobei der Alkylrest bis zu 8 Kohlenstoffatome enthält, dadurch gekennzeichnet, daß man als Ausgangsmaterial ein Produkt der Formel (V) verwendet, worin R wie in Anspruch 1 defiliert ist,und ein Produkt der Formel (IV) verwendet, worin X und Z wie in Anspruch 1 definiert sind und Y wie vorstehend defiliert ist.

5. Verfahren gemäß Anspruch 3, dadurch gekennzeichnet, daß man zu Beginn ein Produkt der Formel (II) verwendet, worin X ein Fluoratom bedeutet.

6. Verfahren gemäß Anspruch 4, dadurch gekennzeichnet, daß man zu Beginn ein Produkt der Formel (IV) verwendet, worin X ein Fluoratom bedeutet.

7. Verfahren gemäß Anspruch 3, dadurch gekennzeichnet, daß man zu Beginn ein Produkt der Formel (II) verwendet, worin X ein Wasserstoffatom bedeutet.

8. Verfahren gemäß Anspruch 4, dadurch gekennzeichnet, daß man zu Beginn ein Produkt der Formel (IV) verwendet, worin X ein Wasserstoffatom bedeutet.

9. Verfahren gemäß einem der Ansprüche 3, 5 oder 7, dadurch gekennzeichnet, daß man zu Beginn ein Produkt der Formel (II) verwendet, worin R einen gesättigten, linearen oder verzweigten Alkylrest mit bis zu 4 Kohlenstoffatomen bedeutet.

10. Verfahren gemäß einem der Ansprüche 4, 6 oder 8, dadurch gekennzeichnet, daß man zu Beginn ein Produkt der Formel (V) verwendet, worin R einen gesättigten, linearen oder verzweigten Alkylrest mit bis zu 4 Kohlenstoffatomen bedeutet.

11. Verfahren gemäß einem der Ansprüche 3, 5, 7 oder 9, dadurch gekennzeichnet, daß man zu Beginn ein Produkt der Formel (III) verwendet, worin R ein Wasserstoffatom bedeutet.

12. Verfahren gemäß einem der Ansprüche 4, 6, 8 oder 10, dadurch gekennzeichnet, daß man zu Beginn ein Produkt der Formel (IV) verwendet, worin R ein Wasserstoffatom bedeutet.

13. Verfahren gemäß einem der Ansprüche 3, 5, 7, 9 oder 11, dadurch gekennzeichnet, daß man zu Beginn ein Produkt der Formel (III) verwendet, worin Y einen Rest $CH_3$, einen Rest $-(CH_2)_m-O-CH_3$, worin m die Zahl 0 oder 1 ist, oder einen Rest $-CH_2-CH=CH_2$ bedeutet.

14. Verfahren gemäß einem der Ansprüche 4, 6, 8, 10 oder 12, dadurch gekennzeichnet, daß man zu Beginn ein Produkt der Formel (IV) verwendet, worin Y einen Rest $CH_3$, einen Rest $-(CH_2)_m-O-CH_3$, worin m die Zahl 0 oder 1 ist, oder einen Rest $-CH_2-CH=CH_2$ bedeutet.

**Patentansprüche für den Vertragsstaat : GR**

1. Verfahren zur Herstellung der Verbindungen der Formel (I)

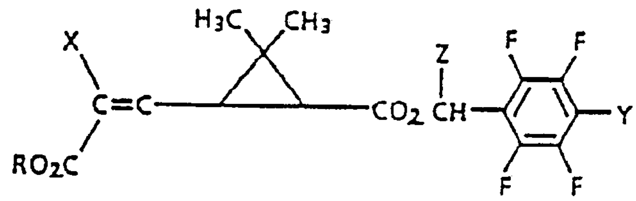

(I)

worin :

– X ein Wasserstoff-, Fluor-, Chlor- oder Bromatom bedeutet,

– R einen linearen, verzweigten oder cyclischen, gesättigten oder ungesättigten, gegebenenfalls substituierten Alkylrest mit bis zu 8 Kohlenstoffatomen, einen gegebenenfalls substituierten Arylrest mit bis zu 14 Kohlenstoffatomen oder einen gegebenenfalls substituierten, heterocyclischen Rest darstellt,

– Z ein Wasserstoffatom, einen Rest $CH_3$, C≡N oder C≡CH bedeutet und

– Y ein Wasserstoffatom, einen Hydroxyrest, einen linearen, verzweigten, gesättigten oder ungesättigten, gebenenfalls substituierten Alkylrest mit bis zu 8 Kohlenstoffatomen, einen Nitrilrest, einen Rest $-(CH_2)_m$-O-alkyl, $-(CH_2)_m$-S-alkyl, $-(CH_2)_m$-N-(alkyl)$_2$, worin m die Zahl 0, 1, 2, 3 oder 4 ist und mit bis zu 12 Kohlenstoffatomen, einen Si(alkyl)$_3$-Rest, wobei Alkyl einen linearen oder verzweigten, gesättigten oder ungesättigten, gegebenenfalls substituierten Alkylrest mit bis zu 8 Kohlenstoffatomen darstellt, einen Rest –O-Aryl– oder $-(CH_2)_m$-Aryl bedeutet, wobei Aryl einen Arylrest mit bis zu 14 Kohlenstoffatomen darstellt, unter allen möglichen stereoisomeren Formen sowie den Gemischen dieser Stereoisomeren, dadurch gekennzeichnet, daß man eine Säure der Formel (II)

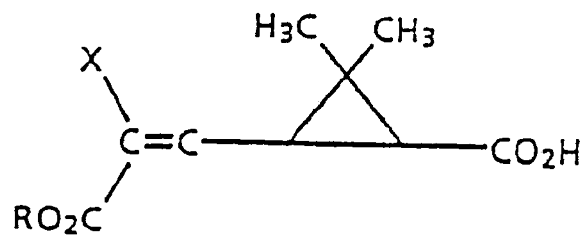

(II)

oder ein funktionelles Derivat dieser Säure, worin X und R die vorstehende Bedeutung haben, der Einwirkung eines Alkohols der Formel (III)

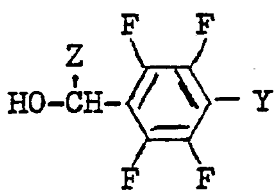

(III)

oder eines funktionellen Derivats dieses Alkohols, worin Y und Z die vorstehende Bedeutung haben, unterwirft, um die entsprechende Verbindung der Formel (I) zu erhalten.

2. Verfahren zur Herstellung der Produkte der Formel (I), wie in Anspruch 1 definiert, dadurch gekennzeichnet, daß man eine Säure der Formel (IV)

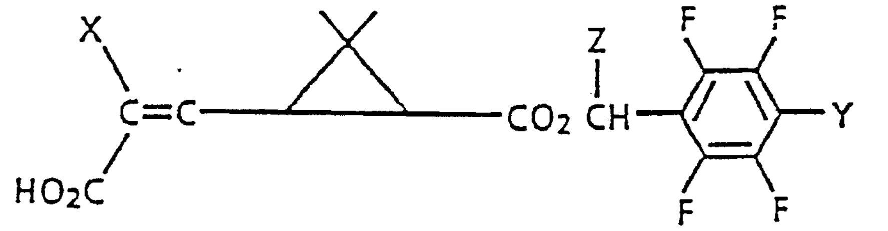

(IV)

worin X, Y und Z die vorstehende Bedeutung haben, der Einwirkung eines Alkohols der Formel V

ROH (V)

worin R die vorstehende Bedeutung hat, unterwirft, um die entsprechende Verbindung der Formel (I) zu erhalten.

3. Verfahren gemäß Anspruch 1 zur Herstellung von Produkten der Formel (I), wie in Anspruch 1 definiert, worin X, R und Z wie in Anspruch 1 defiliert sind und Y ein Wasserstoffatom, einen linearen, verzweigten, gesättigten oder ungesättigten, gegebenenfalls substituierten Alkylrest mit bis zu 8 Kohlenstoffatomen bedeutet, einen Rest $(CH_2)_m$-O-alkyl, einen Rest $(CH_2)_m$-S-alkyl, einen Rest $(CH_2)_m$-$(alkyl)_2$, worin m die Zahl 0, 1, 3 oder 4 ist und mit bis zu 12 Kohlenstoffatomen, oder einen Rest Si-$(alkyl)_3$ bedeutet, wobei der Alkylrest bis zu 8 Kohlenstoffatome enthält, dadurch gekennzeichnet, daß man als Ausgangsmaterial ein Produkt der Formel (II) verwendet, worin X und R wie in Anspruch 1 definiert sind, und ein Produkt der Formel (III) verwendet, worin Z wie in Anspruch 1 definiert ist und Y wie vorstehend definiert ist.

4. Verfahren gemäß Anspruch 2 zur Herstellung von Produkten der Formel (I), wie in Anspruch 1 definiert, worin X, R und Z wie in Anspruch 1 definiert sind und Y ein Wasserstoffatom, einen linearen, verzweigten, gesättigten oder ungesättigten, gegebenenfalls substituierten Alkylrest mit bis zu 8 Kohlenstoffatomen bedeutet, einen Rest $(CH_2)_m$-O-alkyl, einen Rest $(CH_2)_m$-S-alkyl, einen Rest $(CH_2)_m$-$(alkyl)_2$ bedeutet, worin m die Zahl 0, 1, 3 oder 4 ist und mit bis zu 12 Kohlenstoffatomen, oder einen Rest Si-$(alkyl)_3$ bedeutet, wobei der Alkylrest bis zu 8 Kohlenstoffatome enthält, dadurch gekennzeichnet, daß man als Ausgangsmaterial ein Produkt der Formel (V) verwendet, worin R wie in Anspruch 1 definiert ist, und ein Produkt der Formel (IV) verwendet, worin X und Z wie in Anspruch 1 definiert sind und Y wie vorstehend definiert ist.

5. Verfahren gemäß Anspruch 3, dadurch gekennzeichnet, daß man zu Beginn ein Produkt der Formel (II) verwendet, worin X ein Fluoratom bedeutet.

6. Verfahren gemäß Anspruch 4, dadurch gekennzeichnet, daß man zu Beginn ein Produkt der Formel (IV) verwendet, worin X ein Fluoratom bedeutet.

7. Verfahren gemäß Anspruch 3, dadurch gekennzeichnet, daß man zu Beginn ein Produkt der Formel (II) verwendet, worin X ein Wasserstoffatom bedeutet.

8. Verfahren gemäß Anspruch 4, dadurch gekennzeichnet, daß man zu Beginn ein Produkt der Formel (IV) verwendet, worin X ein Wasserstoffatom bedeutet.

9. Verfahren gemäß einem der Ansprüche 3, 5 oder 7, dadurch gekennzeichnet, daß man zu Beginn ein Produkt der Formel (II) verwendet, worin R einen gesättigten, linearen oder verzweigten Alkylrest mit bis zu 4 Kohlenstoffatomen bedeutet.

10. Verfahren gemäß einem der Ansprüche 4, 6 oder 8, dadurch gekennzeichnet, daß man zu Beginn ein Produkt der Formel (V) verwendet, worin R einen gesättigten, linearen oder verzweigten Alkylrest mit bis zu 4 Kohlenstoffatomen bedeutet.

11. Verfahren gemäß einem der Ansprüche 3, 5, 7 oder 9, dadurch gekennzeichnet, daß man zu Beginn ein Produkt der Formel (III) verwendet, worin R ein Wasserstoffatom bedeutet.

12. Verfahren gemäß einem der Ansprüche 4, 6, 8 oder 10, dadurch gekennzeichnet, daß man zu Beginn ein Produkt der Formel (IV) verwendet, worin R ein Wasserstoffatom bedeutet.

13. Verfahren gemäß einem der Ansprüche 3, 5, 7, 9 oder 11, dadurch gekennzeichnet, daß man zu Beginn ein Produkt der Formel (III) verwendet, worin Y einen Rest $CH_3$, einen Rest $-(CH_2)_m$-O-$CH_3$, worin m die Zahl 0 oder 1 ist, oder einen Rest $-CH_2$-CH=$CH_2$ bedeutet.

14. Verfahren gemäß einem der Ansprüche 4, 6, 8, 10 oder 12, dadurch gekennzeichnet, daß man zu Beginn ein Produkt der Formel (IV) verwendet, worin Y einen Rest $CH_3$, einen Rest $-(CH_2)_m$-O-$CH_3$, worin m die Zahl 0 oder 1 ist, oder einen Rest $-CH_2$-CH=$CH_2$ bedeutet.

15. Verfahren gemäß Anspruch 1, dadurch gekennzeichnet, daß man zu Beginn die Verbindungen der Formel (II) und (III) derart wählt, daß man herstellt:

1R-[1α,3α-(Z)]-2,2-Dimethyl-3-(3-methoxy-3-oxo-1-propenyl)-cyclopropan-carbonsäure-(4-methoxy-2,

3,5,6-tetrafluorphenyl)-methylester ;
1R-[1α,3α-(E)]-2,2-Dimethyl-3-(2-fluor-3-ethoxy-3-oxo-1-propenyl)-cyclopropan-carbonsäure-(4-methyl-2,3,5,6-tetrafluorphenyl)-methylester ;
1R-[1α,3α-(E)]-2,2-Dimethyl-3-[3-(1,1-dimethylethoxy)-3-oxo-2-fluor-1-propenyl]-cyclopropancarbonsäure-(4-methyl-2,3,5,6-tetrafluorphenyl)-methylester ;
[1R-(1α,3α)-(E)]-2,2-Dimethyl-3-(3-ethoxy-2-fluor-3-oxo-1-propenyl)-cyclopropan-carbonsäure-[4-(2-propenyl)-2,3,5,6-tetrafluorphenyl]-methylester ;
1R-[1α,3α-(E)]-2,2-Dimethyl-3-[2-fluor-3-(1,1-dimethylethoxy-3-oxo-1-propenyl]-cyclopropan-carbonsäure-(4-methoxy-2,3,5,6-tetrafluorphenyl)-methylester ;
1R-[1α,3α-(E)]-2,2-Dimethyl-3-(2-fluor-3-ethoxy-3-oxo-1-propenyl)-cyclopropan-carbonsäure-(4-methoxy-2,3,5,6-tetrafluorphenyl)-methylester ;
[1R-[1α,3α-(E)]]-2,2-Dimethyl-3-(2-fluor-3-ethoxy-3-oxo-1-propenyl)-cyclopropan-carbonsäure-[(4-methoxymethyl-2,3,5,6-tetrafluor)-phenyl]-methylester ;
[1R-[1α,3α-(E)]]-2,2-Dimethyl-3-[3-(1,1-dimethylethoxy-2-fluor-3-oxo-1-propenyl)-cyclopropan-carbonsäure-[(4-methoxymethyl-2,3,5,6-tetrafluor)-phenyl]-methylester ;
[1R-[1α,3α-(E)]]-2,2-Dimethyl-3-[2-fluor-3-methoxy-3-oxo-1-propenyl)-cyclopropan-carbonsäure-[(4-methoxymethyl-2,3,5,6-tetrafluor)-phenyl]-methylester.

16. Verfahren gemäß Anspruch 2, dadurch gekennzeichnet, daß man zu Beginn die Verbindungen der Formel (IV) und (V) derart wählt, daß man herstellt :
1R[1α,3α-(Z)]-2,2-Dimethyl-3-(3-methoxy-3-oxo-1-propenyl)-cyclopropan-carbonsäure-(4-methoxy-2,3,5,6-tetrafluorphenyl)-methylester ;
1R-[1α,3α-(E)]-2,2-Dimethyl-3-(2-fluor-3-ethoxy-3-oxo-1-propenyl)-cyclopropan-carbonsäure-(4-methyl-2,3,5,6-tetrafluorphenyl)-methylester ;
1R-[1α,3α-(E)]-2,2-Dimethyl-3-[3-(1,1-dimethylethoxy)-3-oxo-2-fluor-1-propenyl]-cyclopropan-carbonsäure-(4-methyl-2,3,5,6-tetrafluorphenyl)-methylester ;
[1R-(1α,3α)-(E)]-2,2-Dimethyl-3-(3-ethoxy-2-fluor-3-oxo-1-propenyl)-cyclopropan-carbonsäure-[4-(2-propenyl)-2,3,5,6-tetrafluorphenyl]-methylester ;
1R-(1α,3α)-(E)]-2,2-Dimethyl-3-[2-fluor-3-(1,1-dimethylethoxy-3-oxo-1-propenyl]-cyclopropan-carbonsäure-(4-methoxy-2,3,5,6-tetrafluorphenyl)-methylester ;
1R-[1α,3α-(E)]-2,2-Dimethyl-3-(2-fluor-3-ethoxy-3-oxo-1-propenyl)-cyclopropan-carbonsäure-(4-methoxy-2,3,5,6-tetrafluorphenyl)-methylester ;
[1R-[1α,3α-(E)]]-2,2-Dimethyl-3-(2-fluor-3-ethoxy-3-oxo-1-propenyl)-cyclopropan-carbonsäure-[(4-methoxymethyl-2,3,5,6-tetrafluor)-phenyl]-methylester ;
[1R-[1α,3α-(E)]]-2,2-Dimethyl-3-[3-(1,1-dimethylethoxy-2-fluor-3-oxo-1-propenyl]-cyclopropan-carbonsäure-[(4-methoxymethyl-2,3,5,6-tetrafluor)-phenyl]-methylester ;
[1R-[1α,3α-(E)]-2,2-Dimethyl-3-[2-fluor-3-methoxy-3-oxo-1-propenyl)-cyclopropan-carbonsäure-[(4-methoxymethyl-2,3,5,6-tetrafluor)-phenyl]-methylester.

17. Zusammensetzungen, bestimmt zur Bekämpfung von Pflanzenparasiten, Parasiten in Räumlichkeiten und Parasiten bei Warmblütern, dadurch gekennzeichnet, daß sie als aktives Prinzip mindestens eine Verbindung, definiert in einem der Ansprüche 1 bis 14, enthalten.

18. Insektizide Zusammensetzungen, dadurch gekennzeichnet, daß sie als aktives Prinzip mindestens eine Verbindung, definiert in einem der Ansprüche 1 bis 14, enthalten.

19. Akarizide Zusammensetzungen, dadurch gekennzeichnet, daß sie als aktives Prinzip mindestens eine Verbindung, defiliert In einem der Ansprüche 1 bis 14, enthalten.

20. Zusammensetzungen mit insektizider, akarizider oder nematizider Aktivität, dadurch gekennzeichnet, daß sie als aktives Material einesteils zumindest eine der Verbindungen der Formel (I) und anderenteils zumindest einen Pyrethrinoidester, ausgewählt aus der Gruppe, bestehend aus den Estern der Allethrolone, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der Chrysanthemumsäuren, den Estern des 5-Benzyl-3-furylmethylalkohols der 2,2-Dimethyl-3-(2-oxo-3-tetrahydrothiophenylidenmethyl)-cyclopropan-1-carbonsäuren, den Estern des 3-Phenoxybenzylalkohols und der α-Cyano-3-phenoxybenzylalkohole der 2,2-Dimethyl-3-(2,2-dichlorvinyl)-cyclopropan-1-carbonsäuren, den Estern der α-Cyano-3-phenoxybenzylalkohole der 2,2-Dimethyl-3-(2,2-dibromvinyl)-cyclopropan-1-carbonsäuren,. den Estern des 3-Phenoxybenzylalkohols der 2-p-Chlorphenyl-2-isopropylessigsäuren, den Estern der Allethrolone, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der 2,2-Dimethyl-3-(1,2,2,2-tetrahaloethyl)-cyclopropan-1-carbonsäuren, enthalten, worin "halo" ein Fluor-, Chlor- oder Bromatom wiedergibt, mit der Maßgabe, daß die Verbindungen I in sämtlichen ihrer möglichen stereoisomeren Formen ebenso wie die Verbindungen der Säuren und Alkohole der vorstehenden Pyrethrinoidester vorliegen können.

21. Zusammensetzungen gemäß einem der Ansprüche 17 bis 20, dadurch gekennzeichnet, daß sie als aktives Prinzip mindestens eines der Verbindungen der Formel (I), wie sie in Anspruch 3 definiert ist, enthalten.

22. Zusammensetzungen gemäß einem der Ansprüche 17 bis 20, dadurch gekennzeichnet, daß sie als aktives Prinzip mindestens eine der Verbindungen der Formel (I), wie sie in Anspruch 4 defiliert ist, enthalten.